# EUROPEAN PATENT APPLICATION

(11) **EP 4 368 189 A1**
(43) Date of publication of application: **15.05.2024**
(21) Application number: 22206337.2
(22) Date of filing: 09.11.2022
(51) Int. Cl.: A61K 35/00, A61K 35/02, A61K 39/00, A61K 38/04

(54) **PEPTIDES AND COMBINATIONS OF PEPTIDES FOR USE IN IMMUNOTHERAPY AGAINST ACUTE MYELOID LEUKEMIA (AML) AND OTHER HEMATOLOGICAL NEOPLASMS**

(71) Applicant: Eberhard Karls Universität Tübingen, Medizinische Fakultät, 72074 Tübingen (DE)
(72) Inventor: Walz, Juliane, 72074 Tübingen (DE); Nelde, Annika, 72070 Tübingen (DE); Rammensee, Hans-Georg, 72070 Unterjesingen (DE); Schuster, Heiko, 71296 Heimsheim (DE)
(74) Representative: Witte, Weller & Partner Patentanwälte mbB

(57) **Abstract**

The present invention relates to peptides, proteins, nucleic acids and cells for use in immunotherapeutic methods. In particular, the present invention relates to the immunotherapy of cancer, in particular of hematological neoplasms, such as acute myeloid leukemia (AML). The present invention furthermore relates to tumor-associated T-cell peptide epitopes that can for example serve as active pharmaceutical ingredients of vaccine compositions that stimulate anti-tumor immune responses, or to stimulate T cells *ex vivo* and transfer into patients. Peptides bound to molecules of the major histocompatibility complex (MHC), or peptides as such, can also be targets of antibodies, soluble T-cell receptors, and other binding molecules.

## Description

The present invention relates to peptides, proteins, nucleic acids and cells for use in immunotherapeutic methods. In particular, the present invention relates to the immunotherapy of cancer, in particular of hematological neoplasms, such as acute myeloid leukemia (AML). The present invention furthermore relates to tumor-associated T-cell peptide epitopes that can for example serve as active pharmaceutical ingredients of vaccine compositions that stimulate anti-tumor immune responses, or to stimulate T cells *ex vivo* and transfer into patients. Peptides bound to molecules of the major histocompatibility complex (MHC), or peptides as such, can also be targets of antibodies, soluble T-cell receptors, and other binding molecules.

The present invention relates to several novel peptide sequences and their variants that can be used in vaccine compositions for eliciting anti-tumor immune responses, in particular against hematological neoplasms, such as acute myeloid leukemia (AML), or as targets for the development of pharmaceutically/immunologically active compounds and cells.

### FIELD OF THE INVENTION

The present invention relates to the field of molecular biology or medicine, more particular to the field of molecular immunology.

### BACKGROUND OF THE INVENTION

Hematologic neoplasms comprise multiple malignant diseases derived from cells of myeloid or lymphocytic hematopoietic lineages. Hence, the classification of these disorders is primarily based on the hematopoietic lineage into lymphoid and myeloid neoplasms. Leukemias are among the most important representatives of hematological neoplasms. Of these, acute myeloid leukemia (AML) in particular is of considerable clinical importance.

Acute myeloid leukemia (AML) is a cancer of the myeloid line of blood cells, characterized by the rapid growth of abnormal cells that build up in the bone marrow and blood and interfere with normal blood cell production. Symptoms may include feeling tired, shortness of breath, easy bruising and bleeding, and increased risk of infection. Occasionally, spread may occur to the brain, skin, or gums. As an acute leukemia, AML progresses rapidly, and is typically fatal within weeks or months if left untreated.

Risk factors of AML include smoking, previous chemotherapy or radiation therapy, myelodysplastic syndrome, and exposure to the chemical benzene. The underlying mechanism involves replacement of normal bone marrow with leukemia cells, which results in a drop in red blood cells, platelets, and normal white blood cells. Diagnosis is generally based on bone marrow aspiration and specific blood tests. AML has several subtypes for which treatments and outcomes may vary.

AML is a rare disease with an incidence of about three new cases/100,000 per year. In Germany, there are about 3,600 new cases per year. It is predominantly a disease of older age; the median age at diagnosis is 63 years. AML accounts for about 80% of all acute leukemias in adults. Men are affected slightly more often than women (1.4:1 ratio). In childhood, only 15 to 20% of patients with acute leukemia have AML. However, the rare acute leukemia of neonatal age is usually AML.

The first-line treatment of AML is typically chemotherapy, with the aim of inducing remission. People may then go on to receive additional chemotherapy, radiation therapy, or a stem cell transplant.

The major challenge in curing AML is the elimination of leukemia stem and progenitor cells (LPCs), therapy-resistant cells that persist after standard therapy and are considered the major cause of leukemic relapses. Therefore, long-term survival of AML patients remains exceptionally poor as the majority of patients relapse despite achieving remission.

T cell-based immunotherapy concepts such as checkpoint inhibitors, CAR T cells, adoptive T cell transfer and vaccination strategies have gained increasing importance in the treatment of hematologic neoplasms in recent years. The main prerequisite for the development of antigen-specific immunotherapy concepts is the identification of suitable targets that show a natural, high-frequency, and tumor-exclusive presentation on the cell surface of tumor cells and are recognized by the patients' immune system. Such targets are presented either by HLA-independent molecules or by HLA class I- and HLA class II molecules on the surface of tumor cells.

For AML, very few target antigens have been described for the development of immunotherapies. For the HLA-independent surface antigens, only CD33, CD123, and FLT3 have been able to achieve clinical relevance with the development of antibodies and CAR-T cells. For vaccination approaches and adoptive TCR-based T cell transfer, these surface antigens, which are not presented via HLA molecules, are not of relevance. Also, for the HLA-presented targets, only very few immunogenic AML-associated antigens have been described so far (WT1, PRAME, NY-ESO-1, hTERT). Although initial clinical studies have shown encouraging results in terms of *in vivo* immunogenicity and clinical response in individual patients, these antigens are mostly restricted to one HLA allotype and have not yet demonstrated therapeutic relevance in more advanced clinical studies.

The widely used strategy for identification of tumor antigens based on gene expression analysis and *in silico* prediction of the resulting HLA ligands is critically hindered by the biased correlation of gene expression and HLA-restricted antigen presentation. That is, it cannot be predicted from gene expression whether an antigen is truly presented on the cell surface of tumor cells.

This circumstance underlines the need for the identification of new path-ophysiologically relevant tumor antigens of hematologic neoplasms in general and AML in particular.

It is, therefore, an object underlying the invention to provide tumor-associated T-cell peptide epitopes, which can be used to develop improved medicaments and methods for the diagnosis, prophylaxis and treatment of hematologic neoplasms such as acute myeloid leukemia (AML). In particular, a pharmaceutical composition, such as a peptide-based vaccine should be provided, which can be used for the diagnosis and/or prevention and/or long-lasting and more effective treatment of hematologic neoplasms.

### SUMMARY OF THE INVENTION

The present invention provides a peptide comprising an amino acid sequence selected from the group consisting of SEQ ID NO: 1 to SEQ ID NO: 32, and variant sequences thereof, which are at least 88% homologous to SEQ ID NO: 1 to SEQ ID NO: 32, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

The inventors now satisfy for the first time the need to provide new target antigens for the development of immunotherapies against hematologic neoplasms such as acute myeloid leukemia (AML). In doing so, the inventors have moved away from the previously common strategy of identifying tumor antigens based on gene expression analysis and *in silico* predictions of the resulting HLA ligands.

Furthermore, the inventors recognized that a key reason for the sobering results to date of immunotherapeutic approaches to the treatment of AML and other hematologic neoplasms is that these known therapies focus on AML blasts and do not target LPC-specific antigens. The inventors, however, have taken into account the peculiarity of AML and other hematologic neoplasms, namely that LPC are resistant to standard therapies and therefore very often lead to relapse.

Therefore, in developing the peptides according to the invention, the inventors have identified and used such target structures that are presented not only by AML blasts but also, to a particular extent, by AML progenitor and stem cells or LPC, respectively. The inventors, therefore, applied a novel strategy. All peptides are found at high frequency and exclusively in the immunopeptidome of AML patients, but never in the immunopeptidome of healthy reference subjects. The peptide(s) according to the invention are therefore particularly effective and have few side effects.

As a result of this approach, the inventors were able to identify 15 different hematological neoplasms- and AML-specific MHC/HLA class II peptides comprising amino acid sequences of SEQ ID NO: 1 to SEQ ID NO: 15. Such MHC/HLA class II peptides can be used in any hematological neoplasms patient independent of its MHC/HLA allotype.

The inventors further identified 17 different hematological neoplasms- and AML-specific MHC/HLA class I peptides comprising the amino acid sequences of SEQ ID NO: 16 to SEQ ID NO: 32. These are the MHC/HLA class I presented peptides of HLA allotypes A*11 (SEQ ID NO: 16), A*03 (SEQ ID NO: 17), A*02 (SEQ ID NO: 18 to SEQ ID NO: 20), A*01 (SEQ ID NO: 21 to SEQ ID NO: 23), B*07 (SEQ ID NO: 24 to SEQ ID NO: 26), C*07 (SEQ ID NO: 24 to SEQ ID NO: 26), C*07 (SEQ ID NO: 27 to SEQ ID NO: 29), and B*08 (SEQ ID NO: 30 to SEQ ID NO: 32).

All selected peptides were identified with high frequency and exclusively in the cohort immunopeptidome, whereas they were never identified in the healthy controls.

The following table 1 shows the MHC/HLA class II peptides according to the invention.

**Table 1: MHC/HLA class II peptides according to the invention.**

| | |
|---|---|
| KLKKMWKSPNGTIQNILGGTVF | SEQ ID NO: 1 |
| DRVKLGTDYRLHLSPV | SEQ ID NO: 2 |
| ETLHKFASKPASEFVK | SEQ ID NO: 3 |
| PHRKKKPFIEKKKAVSFHLVHR | SEQ ID NO: 4 |
| SPGPFPFIQDNISFYA | SEQ ID NO: 5 |
| IGSYIERDVTPAIM | SEQ ID NO: 6 |
| SKPGVIFLTKKGRRF | SEQ ID NO: 7 |
| DRQQMEALTRYLRAAL | SEQ ID NO: 8 |
| GNQLFRINEANQLMQ | SEQ ID NO: 9 |
| LGQEVALNANTKNQKIR | SEQ ID NO: 10 |
| NGRTFHLTRTLTVK | SEQ ID NO: 11 |
| LDTMRQIQVFEDEPAR | SEQ ID NO: 12 |
| VVGYALDYNEYFRDL | SEQ ID NO: 13 |
| KHLHYWFVESQKDPEN | SEQ ID NO: 14 |
| ERPEWIHVDSRPF | SEQ ID NO: 15 |

The following table 2 shows the MHC/HLA class I peptides according to the invention.

**Table 2: MHC/HLA class I peptides according to the invention.**

| MHC class I allotype | | | |
|---|---|---|---|
| A*11 | AVEEVSLRK (SEQ ID NO: 16) | | |
| A*03 | LAVEEVSLR (SEQ ID NO: 17) | | |
| A*02 | SLLEADPFL (SEQ ID NO: 18) | IILDALPQL (SEQ ID NO: 19) | ILNDVAMFL (SEQ ID NO: 20) |
| A*01 | DIDTRSEFY (SEQ ID NO: 21) | FSEYFGAIY (SEQ ID NO: 22) | YLDGRLEPLY (SEQ ID NO: 23) |
| B*07 | APESKHKSSL (SEQ ID NO: 24) | APGLHLEL (SEQ ID NO: 25) | APTIVGKSSL (SEQ ID NO: 26) |
| C*07 | AYHELAQVY (SEQ ID NO: 27) | SRPPLLGF (SEQ ID NO: 28) | IYSGYIFDY (SEQ ID NO: 29) |
| B*08 | DLDTRVAL (SEQ ID NO: 30) | APTPRIKAEL (SEQ ID NO: 31) | EGYGRYLDL (SEQ ID NO: 32) |

According to the findings of the inventors, the following peptides or neoantigens are of particular interest and therefore particularly preferred: KLKKMWKSPNGTIQNILGGTVF (SEQ ID NO: 1); AVEEVSLRK (SEQ ID NO: 16), and LAVEEVSLR (SEQ ID NO: 17). Of interest and also preferred are the following (non-mutated) peptides: DRVKLGTDYRLHLSPV (SEQ ID NO: 2), ETLHKFASKPASEFVK (SEQ ID NO: 3), PHRKKPFIEKKKAVSFHLVHR (SEQ ID NO: 4), SPGPFPFIQDNISFYA (SEQ ID NO: 5), IGSYIERDVTPAIM (SEQ ID NO: 6), SKPGVIFLTKKGRRF (SEQ ID NO: 7), DRQQMEALTRYLRAAL (SEQ ID NO: 8), SLLEADPFL (SEQ ID NO: 18), DIDTRSEFY (SEQ ID NO: 21), APESKHKSSL (SEQ ID NO: 24), APGLHLEL (SEQ ID NO: 25), and AYHELAQVY (SEQ ID NO: 27). The peptides are particularly interesting because they are naturally presented, are tumor-exclusive, i.e., never presented on healthy tissue, and are recognized by T cells.

In the event of discrepancies between the sequences specified in tables 1 and 2 and those specified in the sequence listing, the information in the tables takes precedence and applies.

The term "peptide" is used herein to designate a series of amino acid residues, connected one to the other typically by peptide bonds between the alpha-amino and carbonyl groups of the adjacent amino acids. The peptides are preferably between 7 and 12 amino acids in length, further preferably between 8 and 11, but can be as long as 5, 6, 7, 8, 9, 10, 11, 12, 13, 14, 15, 16, 17, 18, 19, 20, 21, 22 or longer.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "peptide" shall also include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 15 amino acids in length.

The term "peptide" shall also include "polypeptide". The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an MHC molecule in substantially the same way as a peptide consisting of the given amino acid sequence in consisting of SEQ ID NO: 1 to SEQ ID NO: 32. For example, a peptide may be modified so that it at least maintains, if not improves, the ability to interact with and bind to the binding groove of a suitable MHC molecule and in that way, it at least maintains, if not improves, the ability to bind to the TCR of activated T cells.

The original (unmodified) peptides as disclosed herein can be modified by the substitution of one or more residues at different, possibly selective, sites within the peptide chain, if not otherwise stated. Preferably those substitutions are located at the end of the amino acid chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions". Conservative substitutions are herein defined as exchanges within one of the following five groups: Group 1-small aliphatic, nonpolar or slightly polar residues (Ala, Ser, Thr, Pro, GIY); Group 2-polar, negatively charged residues and their amides (Asp, Asn, Glu, Gln); Group 3-polar, positively charged residues (His, Arg, Lys); Group 4-large, aliphatic, nonpolar residues (Met, Leu, Ile, Val, Cys); and Group 5-large, aromatic residues (Phe, Tyr, Trp). Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such "radical" substitutions cannot, however, be dismissed as potentially ineffective since chemical effects are not totally predictable and radical substitutions might well give rise to serendipitous effects not otherwise predictable from simple chemical principles. Of course, such substitutions may involve structures other than the common L-amino acids. Thus, D-amino acids might be substituted for the L-amino acids commonly found in the antigenic peptides of the invention and yet still be encompassed by the disclosure herein. In addition, non-standard amino acids (i.e., other than the common naturally occurring proteinogenic amino acids) may also be used for substitution purposes to produce immunogens and immunogenic polypeptides according to the present invention.

If substitutions at more than one position are found to result in a peptide with substantially equivalent or greater antigenic activity as defined below, then combinations of those substitutions will be tested to determine if the combined substitutions result in additive or synergistic effects on the antigenicity of the peptide. At most, no more than 4 positions within the peptide would be simultaneously substituted.

The amino acid residues that do not substantially contribute to interactions with the T cell receptor can be modified by replacement with other amino acids whose incorporation do not substantially affect T cell reactivity and does not eliminate binding to the relevant MHC.

Longer (elongated) peptides may also be suitable. It is possible that MHC class I epitopes, although usually between 8 and 11 amino acids long, are generated by peptide processing from longer peptides or proteins that include the actual epitope. It is preferred that the residues that flank the actual epitope are residues that do not substantially affect proteolytic cleavage necessary to expose the actual epitope during processing.

The peptides of the invention can be elongated by up to four amino acids, that is 1, 2, 3 or 4 amino acids can be added to either end in any combination between 4:0 and 0:4. Combinations of the elongations according to the invention can be found in Table 3.

**Table 3: Combinations of the elongations of peptides of the invention**

| C-terminus | N-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

| N-terminus | C-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

The amino acids for the elongation/extension can be from the peptides of the original sequence of the protein or any other amino acid(s). The elongation can be used to enhance the stability or solubility of the peptides.

Thus, the epitopes of the present invention may be identical to naturally occurring tumor-associated or tumor-specific epitopes or may include epitopes that differ by no more than four residues from the reference peptide, as long as they have substantially identical antigenic activity.

In an alternative embodiment, the peptide is elongated on either or both sides by more than 4 amino acids, preferably to a total length of up to 30 amino acids. This may lead to MHC class II binding peptides. Binding to MHC class II can be tested by methods known in the art.

Accordingly, the present invention provides peptides and variants of MHC class I epitopes, wherein the peptide or variant has an overall length of between 8 and 100, preferably between 8 and 30, and most preferred between 8 and 14, namely 8, 9, 10, 11, 12, 13, 14 amino acids, in case of the elongated class II binding peptides the length can also be 15, 16, 17, 18, 19, 20, 21 or 22 or 23 amino acids.

Of course, the peptide or variant according to the present invention will have the ability to bind to a molecule of the human major histocompatibility complex (MHC/HLA) class I or II. Binding of a peptide or a variant to an MHC complex may be tested by methods known in the art.

Preferably, when the T cells specific for a peptide according to the present invention are tested against the substituted peptides, the peptide concentration at which the substituted peptides achieve half the maximal increase in lysis relative to background is no more than about 1 mM, preferably no more than about 1 µM, more preferably no more than about 1 nM, and still more preferably no more than about 100 pM, and most preferably no more than about 10 pM. It is also preferred that the substituted peptide be recognized by T cells from more than one individual, at least two, and more preferably three individuals.

A person skilled in the art will be able to assess, whether T cells induced by a variant of a specific peptide will be able to cross-react with the peptide itself (Appay et al., 2006; Colombetti et al., Eur. J. Immunol. 36: 1805-1814 (2006); Fong et al., Proc. Natl. Acad. Sci. U.S.A. 98: 8809-8814 (2001); Zaremba et al., Cancer Res. 57: 4570-4577 (1997)).

These T cells can subsequently cross-react with cells and kill cells that express a polypeptide that contains the natural amino acid sequence of the cognate peptide as defined in the aspects of the invention. As can be derived from the scientific literature and databases (Rammensee et al., Immunogenetics 50: 213-219 (1999); Godkin et al., Int. Immunol 9: 905-911 (1997)), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. Thus, one skilled in the art would be able to modify the amino acid sequences set forth in SEQ ID NO: 1 to SEQ ID NO: 32, by maintaining the known anchor residues, and would be able to determine whether such variants maintain the ability to bind MHC class I or II molecules. The variants of the present invention retain the ability to bind to the TCR of activated T cells, which can subsequently cross-react with and kill cells that express a polypeptide containing the natural amino acid sequence of the cognate peptide as defined in the aspects of the invention.

In the present invention, the term "homologous" refers to the degree of identity between sequences of two amino acid sequences, i.e., peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each base or amino acid in the Reference Sequence that does not have a corresponding aligned base or amino acid in the Compared Sequence and
(ii) each gap in the Reference Sequence and
(iii) each aligned base or amino acid in the Reference Sequence that is different from an aligned base or amino acid in the Compared Sequence, constitutes a difference and
(iv) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

According to the invention "full-length polypeptide" refers to the source proteins from which the peptides are derived. Full-length polypeptides are also referred to as the source genes/proteins from which the peptides are derived. The source proteins or full-length polypeptides may or may not be highly over-expressed in cancer compared with normal tissues. "Normal tissues" in relation to this invention shall mean either healthy peripheral blood mononuclear cells (PBMC) cells or other normal tissue cells, demonstrating a high degree of tumor association of the source genes. Moreover, the peptides themselves are presented on tumor tissue. "Tumor tissue" in relation to this invention shall mean a sample from a patient suffering from cancer, such as hematologic neoplasms or acute myeloid leukemia (AML). Exemplary "full-length polypeptides" or source proteins are indicated in table 6, column "Source protein".

In an embodiment of the invention the peptide is isolated. The term "isolated" means that the material is removed from its original environment (e.g., the natural environment, if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The peptides and/or nucleotides disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from a cDNA library have been conventionally purified to electrophoretic homogeneity. Purification of starting material or natural material to at least one order of magnitude, preferably two or three orders, and more preferably four or five orders of magnitude is expressly contemplated. Furthermore, a claimed polypeptide which has a purity of preferably 99.999%, or at least 99.99% or 99.9%; and even desirably 99% by weight or greater is expressly encompassed.

The peptides and nucleic acids according to the invention may be in "enriched form". As used herein, the term "enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

Against this background, another subject-matter of the invention is a pharmaceutical composition for the diagnosis and/or prevention and/or treatment of hematological neoplasms, especially acute myeloid leukemia (AML), comprising at least one peptide which binds to class II molecules of the major histocompatibility complex (MHC class II molecules) and/or induces T cells cross-reacting with said peptide, and a pharmaceutically acceptable carrier, wherein said at least one peptide comprises an amino acid sequence which is selected from the group consisting of: SEQ ID NO: 1 to SEQ ID NO: 15 and variant sequences thereof which are at least 88% homologous to SEQ ID NO: 1 to SEQ ID NO: 15, wherein said peptide is not a full-length polypeptide.

Such pharmaceutical composition comprises at least one of the MHC class II peptides according to the invention. It is, therefore, independent from the allotype of the patient.

The features, advantages and embodiments of the peptide(s) according to the invention apply to the pharmaceutical composition according to the invention *mutatis mutandis.*

A "pharmaceutical composition" is a composition suitable for administration to a human being in a medical setting. Preferably, a pharmaceutical composition is sterile and produced according to GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH₂ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

Preferably, the pharmaceutical composition of the present invention is an immunotherapeutic such as a vaccine. It may be administered directly into the patient, into the affected organ or systemically i.d., i.m., s.c., i.p. and i.v., or applied *ex vivo* to cells derived from the patient or a human cell line which are subsequently administered to the patient or used *in vitro* to select a subpopulation of immune cells derived from the patient, which are then re-administered to the patient. If the nucleic acid is administered to cells *in vitro,* it may be useful for the cells to be transfected so as to co-express immune-stimulating cytokines, such as interleukin-2.

The peptide(s) according to the invention may be substantially pure. The peptide(s) may also be combined with an immune-stimulating adjuvant, such as the TLR1/2 ligand XS15. Preliminary work has shown thatXS15 can induce a strong CD8⁺ and Th1CD4⁺ T-cell response *in vivo* after subcutaneous injection in healthy donors and tumor patients for viral, mutated and unmutated peptides in a water-oil emulsion. The peptide(s) may also be used in combination with immune-stimulatory cytokines, or be administered with a suitable delivery system, for example liposomes. The peptide(s) may also be conjugated to a suitable carrier such as keyhole limpet haemocyanin (KLH) or mannan (see WO 95/18145 and Longenecker et al., Ann. N.Y. Acad. Sci. 690:276-291 (1993)). The peptide(s) may also be tagged, may be a fusion protein, or may be a hybrid molecule. The peptides whose sequence is given in the present invention are expected to stimulate CD4 or CD8 T cells. However, stimulation of CD8 T cells is more efficient in the presence of help provided by CD4 T-helper cells. Thus, for MHC Class I epitopes that stimulate CD8 T cells the fusion partner or sections of a hybrid molecule suitably provide epitopes which stimulate CD4-positive T cells. CD4- and CD8-stimulating epitopes are well known in the art and include those identified in the present invention.

According to the invention, "at least one" means 1, 2, 3, 4, 5, 6,7, 8, 9, 10, 11, 12, 13, 14, 15, 16, 17, 18, 19, 20, 21, 22, 23, etc. This ensures coverage of a very high percentage of the world population.

The pharmaceutical composition according to the invention may comprise, as the only active ingredient, the peptide(s) according to the invention. However, in an alternative aspect, it may comprise (an) additional active ingredient(s).

The problem underlying the invention is herewith completely solved.

In an embodiment of the invention the pharmaceutical composition comprises at least 2, preferably at least 3, further preferably at least 4, further preferably at least 5, further preferably at least 6, further preferably at least 7, further preferably at least 8, further preferably at least 9, and highly preferably at least 10 different peptides, each peptide comprising an amino acid sequence which is selected from the group consisting of: SEQ ID NO: 1 to SEQ ID NO: 15 and variant sequences thereof which are at least 88% homologous to SEQ ID NO: 1 to SEQ ID NO: 15, wherein said peptide is not a full-length polypeptide.

This measure has the advantage that, by increasing the number of different MHC class II peptides in the pharmaceutical an increasingly increasing number of individuals will be immunized, regardless of their specific allotype. With 10 different peptides, all population members are covered.

In another embodiment the pharmaceutical composition according to the invention comprises different peptides comprising the following amino acid sequences:
KLKKMWKSPNGTIQNILGGTVF (SEQ ID NO: 1)
DRVKLGTDYRLHLSPV (SEQ ID NO: 2)
ETLHKFASKPASEFVK (SEQ ID NO: 3)
PHRKKKPFIEKKKAVSFHLVHR (SEQ ID NO: 4)
SPGPFPFIQDNISFYA (SEQ ID NO: 5)
IGSYIERDVTPAIM (SEQ ID NO: 6)
SKPGVIFLTKKGRRF (SEQ ID NO: 7)
DRQQMEALTRYLRAAL (SEQ ID NO: 8)
GNQLFRINEANQLMQ (SEQ ID NO: 9)
LGQEVALNANTKNQKIR (SEQ ID NO: 10)
NGRTFHLTRTLTVK (SEQ ID NO: 11)
LDTMRQIQVFEDEPAR (SEQ ID NO: 12)
VVGYALDYNEYFRDL (SEQ ID NO: 13)
KHLHYWFVESQKDPEN (SEQ ID NO: 14)
ERPEWIHVDSRPF (SEQ ID NO: 15).

In still another embodiment of the invention said pharmaceutical composition comprises at least one additional peptide which binds to class I molecules of the major histocompatibility complex (MHC class I molecules) and/or induces T cells cross-reacting with said peptide, wherein said at least one additional peptide comprises an amino acid sequence which is selected from the group consisting of: SEQ ID NO: 16 to SEQ ID NO: 32 and variant sequences thereof which are at least 88% homologous to SEQ ID NO: 16 to SEQ ID NO: 32, wherein said peptide is not a full-length polypeptide.

This measure has the advantage that the pharmaceutical composition of the invention is supplemented with MHC/HLA class I peptides resulting in a (semi-) individualized or personalized composition where the additional peptide(s) is selected in accordance with the MCH/HLA allotype of the patient.

In another embodiment of the invention the at least one additional peptide is selected depending on the MHC class I allotype of the individual to be treated, preferably the at least one additional peptide is selected according to the following table 4, wherein the amino acid sequences of each MHC class I allotype group includes variant sequences which are at least 88% homologous:

**Table 4: Selection of additional peptide(s) depending on the MHC class I allotype of the individual to be treated.**

| MHC class I allotype | Amino acid sequence of peptide |
|---|---|
| A*11 | SEQ ID NO: 16 |
| A*03 | SEQ ID NO: 17 |
| A*02 | SEQ ID NO: 18 to SEA ID NO: 20 |
| A*01 | SEQ ID NO: 21 to SEQ ID NO: 23 |
| B*07 | SEQ ID NO: 24 to SEQ ID NO: 26 |
| C*07 | SEQ ID NO: 27 to SEQ ID NO: 29 |
| B*08 | SEQ ID NO: 30 to SEQ ID NO: 32 |

By this measure, there is provided a pharmaceutical composition comprising, in addition to one or more of the MHC class II peptides according to the invention (SEQ ID NOS: 1-15), one or more further peptides (SEQ ID NOS: 16-32) specifically targeting the MHC allotype of the individual to be treated. This provides a (semi-) personalized pharmaceutical composition that elicits an optimal and maximized immune response in the individual. The pharmaceutical composition is thereby particularly effective in or against a disease of hematological neoplasms, especially acute myeloid leukemia (AML).

Against this background, in an embodiment the peptide according to the invention has the ability to bind to an MHC class I or II molecule, and wherein said peptide, when bound to said MHC, is capable of being recognized by CD4 and/or CD8 T cells. Preferably the amino acid sequence thereof comprises a continuous stretch of amino acids according to any one of SEQ ID NO: 1 to SEQ ID NO: 32.

In yet a further embodiment of the pharmaceutical composition according to the invention it comprises at least 2, preferably at least 3, further preferably at least 4, further preferably at least 5, and highly preferably at least 6 different additional peptides, each additional peptide comprising an amino acid sequence which is selected from the group consisting of: SEQ ID NO: 15 to SEQ ID NO: 32 and variant sequences thereof which are at least 88% homologous to SEQ ID NO: 15 to SEQ ID NO: 32, wherein said additional peptide is not a full-length polypeptide.

As the number of additional peptides increases, the immunostimulatory activity of the pharmaceutical composition of the invention is increasingly enhanced in an allotype-specific manner.

In a preferred embodiment of the pharmaceutical composition according to the invention, the latter contains a total of up to a maximum of 10 different peptides according to the invention. Of these, about 5-7 are MHC class II peptides (SEQ ID NOS: 1-15) and about 3-5 are MHC class I peptides (SEQ ID NOS: 16-32). This provides a "vaccine cocktail" individualized for the patient. The selection of the specific vaccine peptides for the patient-individual cocktail is based on the molecular phenotype of the disease as well as the individual HLA allotype of the patient.

In a still further embodiment of the invention said pharmaceutical composition is a vaccine, preferably a vaccine against hematological neoplasms, further preferably a vaccine against acute myeloid leukemia (AML).

In another embodiment of the invention said pharmaceutical composition further comprises an adjuvant, preferably XS15, further preferably XS15 dissolved in montanide.

XS15 is a TLR1/2 ligand and functions as an immune-stimulating adjuvant. Preliminary work has shown thatXS15 can induce a strong CD8⁺ and Th1CD4⁺ T-cell response *in vivo*; see Rammensee et al. (2019), A new synthetic toll-like receptor 1/2 ligand is an efficient adjuvant for peptide vaccination in a human volunteer. J. Immunother. Cancer. 7(1):307, and Heitmann et al. (2022), A COVID-19 peptide vaccine for the induction of SARS-CoV-2 T cell immunity. Nature 601, 617-622. XS15 is preferably dissolved in in montanide ISA51. The addition of the adjuvant creates optimal conditions for use of the pharmaceutical composition as a vaccine.

Another subject-matter of the invention is an antibody, in particular a soluble or membrane-bound antibody, preferably a monoclonal antibody or fragment thereof, that specifically recognizes the peptide(s) or variant thereof according to the invention, preferably the peptide or variant thereof according to the invention when bound to an MHC molecule.

The term "antibody" or "antibodies" is used herein in a broad sense and includes both polyclonal and monoclonal antibodies. In addition to intact or "full" immunoglobulin molecules, also included in the term "antibodies" are fragments (e.g., CDRs, Fv, Fab and Fc fragments) or polymers of those immunoglobulin molecules and humanized versions of immunoglobulin molecules, as long as they exhibit any of the desired properties, i.e., specifically recognize the peptide(s) or variant thereof according to the invention. Whenever possible, the antibodies of the invention may be purchased from commercial sources. The antibodies of the invention may also be generated using well-known methods.

The features, characteristics, advantages and embodiments disclosed for the pharmaceutical composition and/or the peptide(s) according to the invention apply to the antibody and fragment thereof correspondingly.

Another subject-matter of the invention relates to a T cell receptor, preferably soluble or membrane-bound, or a fragment thereof, that is reactive with an HLA ligand, wherein said ligand is the peptide(s) or variant thereof according to the invention, preferably when bound to an MHC molecule.

The term "T cell receptor" (abbreviated TCR) according to the invention refers to a heterodimeric molecule comprising an alpha polypeptide chain (alpha chain) and a beta polypeptide chain (beta chain), wherein the heterodimeric receptor is capable of binding to a peptide antigen presented by an HLA molecule. The term also includes so-called gamma/delta TCRs.

The features, characteristics, advantages and embodiments disclosed for the peptide(s) and antibody or fragment thereof according to the invention apply to the T cell receptor correspondingly.

A still further subject-matter according to the invention relates to a nucleic acid, encoding for a peptide or variant thereof according to the invention, an antibody or fragment thereof according to the invention, a T cell receptor or fragment thereof according to the invention, optionally linked to a heterologous promoter sequence, or an expression vector expressing said nucleic acid.

The nucleic acid coding for a particular peptide, oligopeptide, or polypeptide may be naturally occurring or it may be synthetically constructed. The nucleic acid (for example a polynucleotide) may be, for example, DNA, cDNA, PNA, RNA or combinations thereof, either single- and/or double- stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing a/the (poly)peptide(s) according to the invention.

As used herein the term "nucleic acid coding for (or encoding) a peptide" refers to a nucleotide sequence coding for the peptide including artificial (man-made) start and stop codons compatible for the biological system the sequence is to be expressed by, for example, a dendritic cell or another cell system useful for the production of TCRs. The term "promoter" means a region of DNA involved in binding of RNA polymerase to initiate transcription.

In an embodiment of the invention the nucleic acid is isolated. The term "isolated" means that the material is removed from its original environment (e.g., the natural environment, if it is naturally occurring). For example, a naturally-occurring polynucleotide or (poly-) peptide present in a living animal is not isolated, but the same polynucleotide or polypeptide, separated from some or all of the coexisting materials in the natural system, is isolated. Such polynucleotides could be part of a vector and/or such polynucleotides or (poly-) peptides could be part of a composition, and still be isolated in that such vector or composition is not part of its natural environment.

The features, characteristics, advantages and embodiments disclosed for the pharmaceutical composition and/or the peptide(s) according to the invention apply to the nucleic acid and expression vector correspondingly.

Another subject-matter of the invention relates to a recombinant host cell comprising the peptide(s) according to the invention, the antibody or fragment thereof according to the invention, the T cell receptor or fragment thereof according to the invention or the nucleic acid or the expression vector according to the invention, wherein said host cell preferably is selected from a mammalian or human cell, further preferably from an antigen presenting cell, such as a dendritic cell, a T cell or an NK cell.

The features, characteristics, advantages and embodiments disclosed for the pharmaceutical composition and/or peptide(s) according to the invention apply to the host cell correspondingly.

A still further subject-matter of the invention relates to an *in vitro* method for producing activated T lymphocytes, the method comprising contacting in vitro T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to the invention.

The activated T cells that are directed against the peptide(s) of the invention are useful in therapy. Thus, a further aspect of the invention provides activated T cells obtainable by the foregoing methods of the invention.

Activated T cells, which are produced by the above method, will selectively recognize a cell that aberrantly expresses a polypeptide that comprises an amino acid sequence of SEQ ID NO: 1 to SEQ ID NO: 32.

The features, characteristics, advantages and embodiments disclosed for the pharmaceutical composition and/or the peptide(s) according to the invention apply to the above method correspondingly.

Another subject-matter according to the invention relates to a pharmaceutical composition comprising at least one active ingredient selected from the group consisting of the peptide according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention, or a conjugated or labelled active ingredient, and a pharmaceutically acceptable carrier, and optionally, pharmaceutically acceptable excipients and/or stabilizers.

The features, characteristics, advantages and embodiments disclosed for the pharmaceutical composition recited at the outset and/or the peptide(s) according to the invention apply to said pharmaceutical composition correspondingly.

Another subject-matter of the invention relates to the peptide(s) according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention for use in medicine, preferably in the diagnosis and/or prevention and/or treatment of cancer, or for use in the manufacture of a medicament against cancer, including hematological neoplasms, preferably a vaccine, further preferably said cancer or hematological neoplasms are acute myeloid leukemia (AML) and other neoplasms or tumors that show an overexpression of a protein from which a peptide SEQ ID NO: 1 to SEQ ID NO: 32 is derived from.

The features, characteristics, advantages and embodiments disclosed for the pharmaceutical composition and/or the peptide(s) according to the invention apply to the above-referenced use correspondingly.

A still further subject-matter according to the invention relates to a kit comprising:
a) a container comprising a pharmaceutical composition according to the invention, the peptide(s) or the variant according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention, in solution or in lyophilized formulation;
b) optionally, a second container containing a diluent or reconstituting solution for the lyophilized formulation;
c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kits of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments disclosed for the pharmaceutical composition and/or the peptide(s) according to the invention apply to the kit correspondingly.

Another subject-matter of the invention relates to a method for producing a personalized anti-cancer pharmaceutical composition, preferably a vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or overpresentation in tumors as compared to normal tissues;
c) selecting at least one peptide from the warehouse that matched a TUMAP identified in said patient; and
d) formulating the personalized vaccine based on step c),
wherein said warehouse comprises a plurality of peptides and/or variant sequences according to the invention.

A "personalized pharmaceutical composition" shall mean specifically tailored therapies for one individual patient that will only be used for therapy in such individual patient, including actively personalized cancer vaccines and adoptive cellular therapies using autologous patient tissue.

As used herein, the term "warehouse" shall refer to the peptides according to the invention that have been pre-screened for immunogenicity and/or over-presentation in a particular tumor type, including hematological neoplasms, preferably acute myeloid leukemia (AML). The warehouse (e.g., in the form of a data-base) is composed of tumor-associated peptides which were highly overexpressed in cancer cells of patients with various HLA-A, HLA-B and HLA-C alleles. It contains MHC class I and MHC class II peptides. In particular, it contains MHC class I A*11, A*03, A*02, A*01, B*07, C*07, and B*08 marker peptides. These peptides allow comparison of the magnitude of T-cell immunity induced by TUMAPS in a quantitative manner and hence allow important conclusion to be drawn on the capacity of the vaccine to elicit anti-tumor responses.

In one exemplary embodiment, the peptides included in the vaccine are identified by: (a) identifying tumor-associated peptides (TUMAPs) presented by a tumor sample from the individual patient by the method as described above; (b) comparing the peptides identified in a) with the warehouse of peptides that have been prescreened for immunogenicity and overpresentation in tumors as compared to corresponding normal tissue; (c) selecting at least one peptide from the warehouse that correlates with a tumor-associated peptide identified in the patient; and (d) optionally, selecting at least one peptide identified *de novo* in (a) confirming its immunogenicity.

Once the peptides for a personalized peptide based pharmaceutical composition, e.g., a vaccine, are selected, the composition is produced. The composition or vaccine preferably is a liquid formulation consisting of the individual peptides dissolved in between 20-40% DMSO, preferably about 30-35% DMSO, such as about 33% DMSO.

The features, characteristics, advantages and embodiments disclosed for the pharmaceutical composition and/or the peptide(s) according to the invention apply to the afore-referenced method correspondingly.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in more detail by referring to the following non-limiting examples and figures:

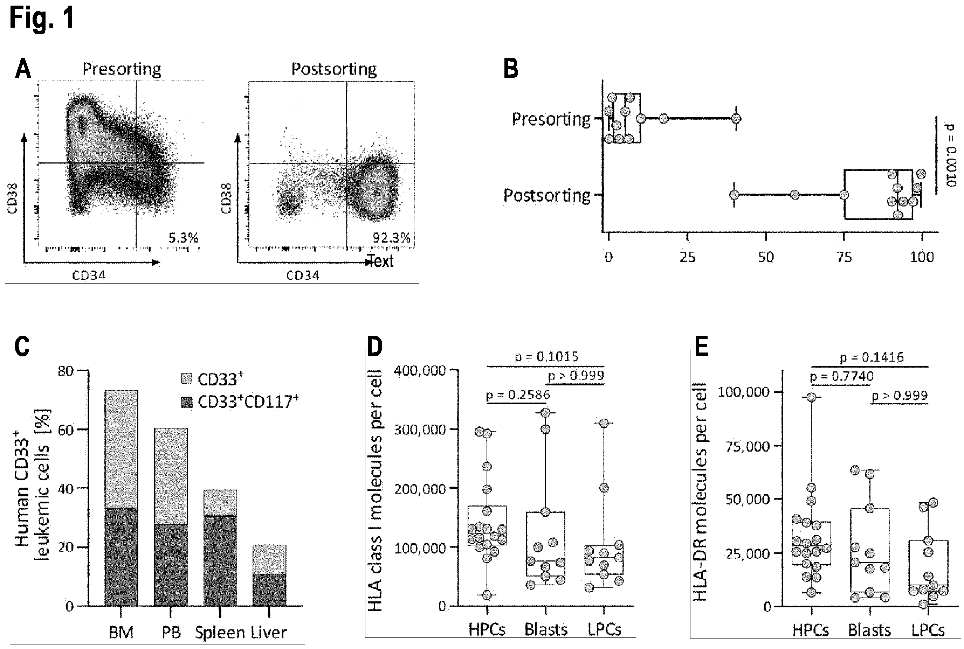

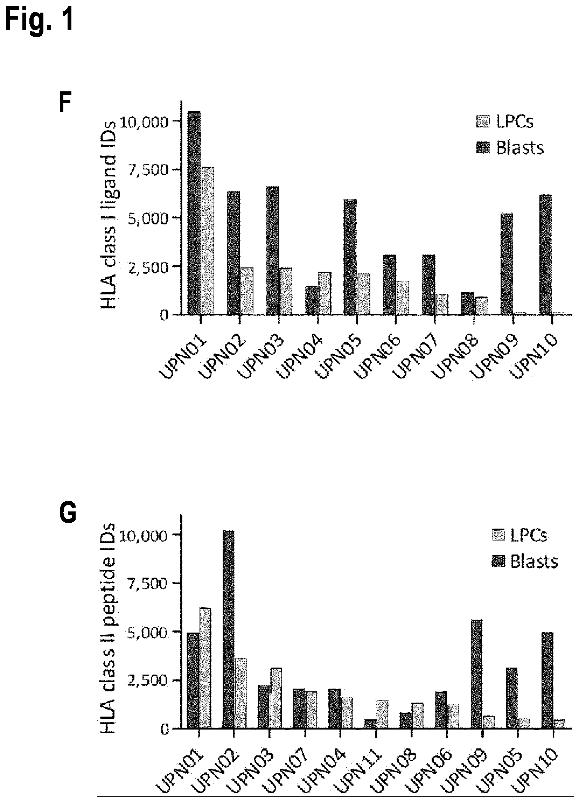

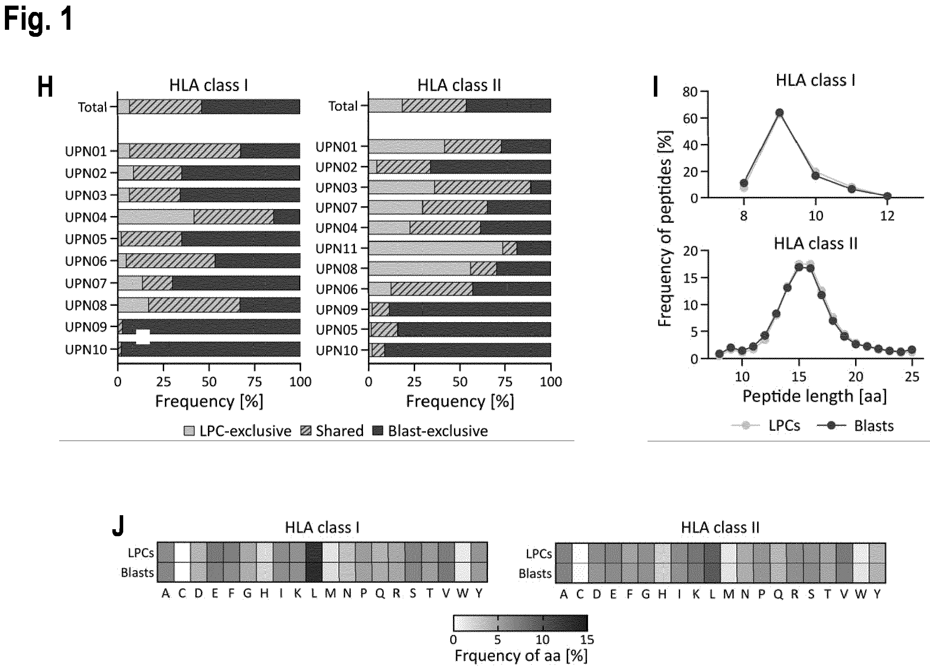

- Fig. 1:: Immunopeptidome analysis of enriched primary CD34⁺CD38⁻ LPCs. (A) Representative flow cytometry analysis of LPC frequencies pre- and post-CD34⁺CD38⁻ enrichment. (B) Frequencies of CD34⁺CD38⁻ LPC in primary AML patient samples (n = 11) pre- and postsorting. Data points represent individual samples. Boxes represent median and 25^{th} to 75^{th} percentiles, whiskers are minimum to maximum, paired Wilcoxon signed rank test. (C) *In vivo* leukemic engraftment of LPCs in NOD/SCID/IL2Rγ^{ull} mice (n = 4). Frequency of human CD33⁺ and CD33⁺CD117⁺ leukemic cells in the bone marrow, peripheral blood, spleen, and liver of NOD/SCID/IL2Rγ^{null} mice 31 weeks after intrafemoral transplantation of 6 × 10⁵ human CD34⁺CD38⁻ LPCs. (D, E) Surface expression of (D) HLA class I and (E) HLA-DR molecules determined by flow cytometry on CD34⁺ HPCs of HVs (n = 18), AML blasts (n = 11) and LPCs (n = 11). Data points represent individual samples. Boxes represent median and 25^{th} to 75^{th} percentiles, whiskers are minimum to maximum, Kruskal-Wallis test. (F, G) Number of (F) HLA class I- and (G) HLA class II-presented peptides on LPCs and autologous blast samples (n = 10 for HLA class I, n = 11 for HLA class II) identified by mass spectrometry. (H) Overlap analysis of HLA class I ligand (left panel) and HLA class II peptide (right panel) identifications of LPCs and autologous blast samples on patient-individual and cohort-wide level. (I) Peptide length distribution of HLA class I ligands (upper panel) and HLA class II peptides (lower panel) in the immunopeptidome of LPCs and autologous blasts. (J) Amino acid distribution within the LPC- and AML blast-derived HLA class I (left panel) and HLA class II (right panel) immunopeptidomes based on the unique peptide identifications in each cohort. Abbreviations: BM, bone marrow; PB, peripheral blood; HPCs, hematopoietic progenitor cells; LPCs, leukemic progenitor cells; IDs, identifications; UPN, uniform patient number; aa, amino acid.

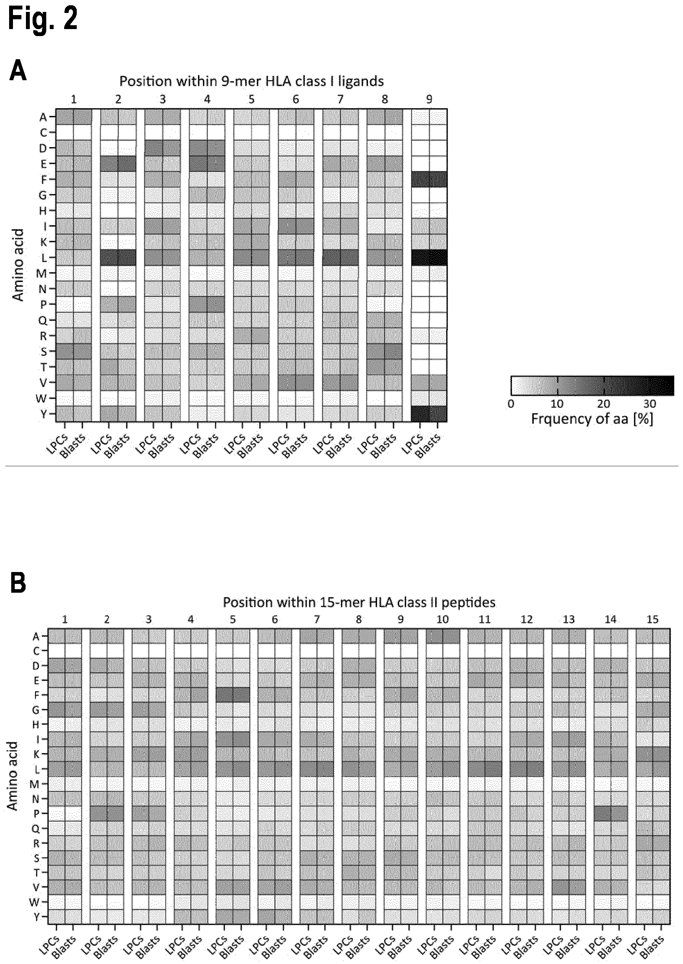

- Fig. 2:: Amino acid distribution within LPC and blast immunopeptidomes. Position-specific amino acid distribution within the LPC- and AML blast-derived (A) HLA class I and (B) HLA class II immunopeptidomes. Thus, the abundance of each amino acids within the 9-mer HLA class I ligands and 15-mer HLA class II peptides was calculated on a sample-by-sample basis for each respective position within the peptides followed by the calculation of the mean amino acid frequency within the LPC and blast cohort, respectively.

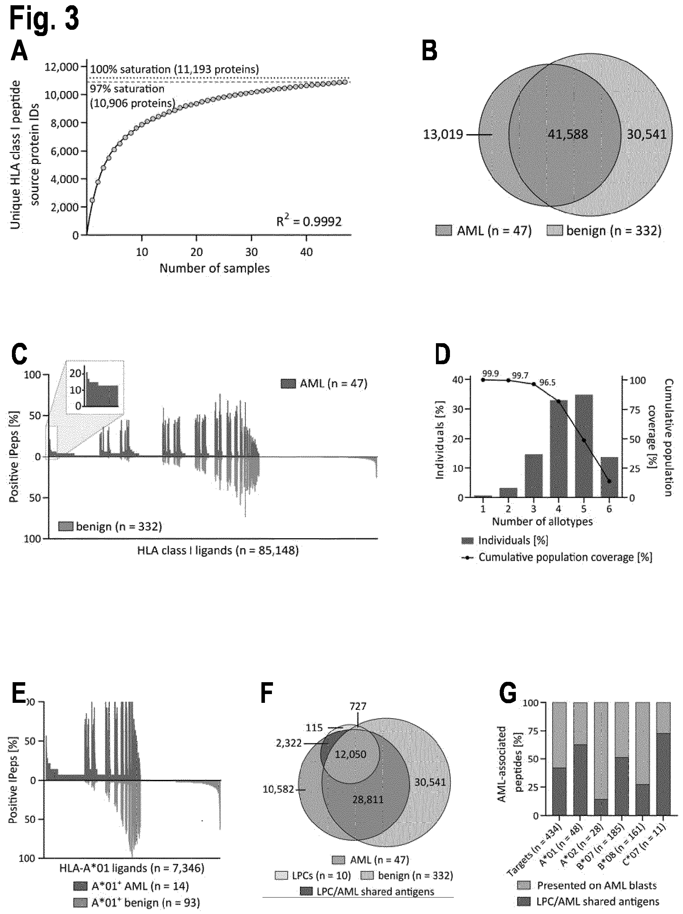

- Fig. 3:: Comparative immunopeptidome profiling identifies AML- and LPC-associated HLA class I antigen targets. (A) Saturation analysis of HLA class I-restricted peptide source proteins of the AML cohort. Number of unique source protein identifications shown as a function of cumulative immunopeptidome analysis of AML samples (n = 47). Exponential regression allowed for the robust calculation of the maximum attainable number of different source protein identifications (dotted lines). The dashed line depicts the source immunoproteome coverage achieved in the AML cohort. (B) Overlap analysis of HLA class I ligand identifications of primary AML samples (n = 47) and benign samples (n = 332). (C) Comparative HLA class I immunopeptidome profiling based on the frequency of HLA-restricted presentation in AML (n = 47) and benign immunopeptidomes (n = 332). Frequencies of positive immunopeptidomes for the respective HLA ligand (x-axis) are indicated on the y-axis. The box on the left and its magnification highlight the subset of AML-associated antigens showing AML-exclusive, high frequent presentation. (D) HLA class I allotype population coverage within the AML cohort compared to the world population. The frequencies of individuals within the world population carrying up to six HLA allotypes (x-axis) of the AML dataset are indicated as grey bars on the left y-axis. The cumulative percentage of population coverage is depicted as black dots on the right y-axis. (E) Representative example of allotype-specific comparative profiling of HLA-A*01 positive samples (AML n = 14, benign n = 93) as described above. (F) Overlap analysis of HLA class I ligand identifications of LPCₑₙᵣ samples (n = 10) with AML (n = 47) and benign samples (n = 332). Overlap analysis and comparative profiling were performed without one hit wonder peptides. (G) Analysis of the proportion of AML-associated HLA class I-presented antigen peptides (all, A*01-, A*02-, B*07-, B*08-, and C*07-restricted targets) that are presented on both AML blasts and LPCs (LPC/AML shared antigens). Abbreviations: IPep, immunopeptidome; n, number.

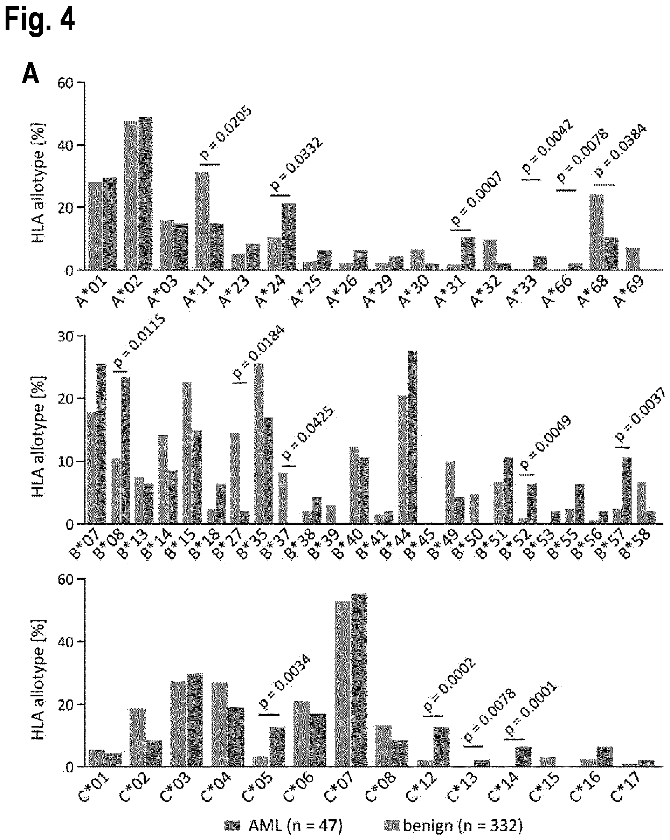

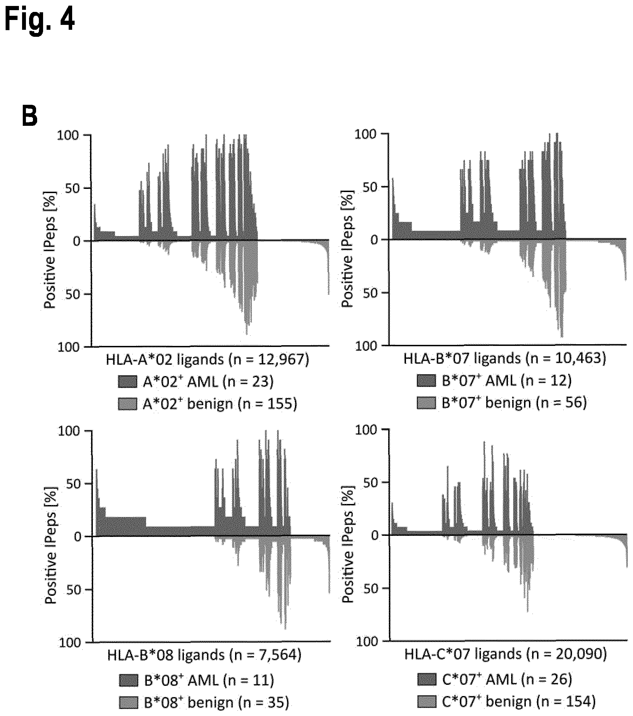

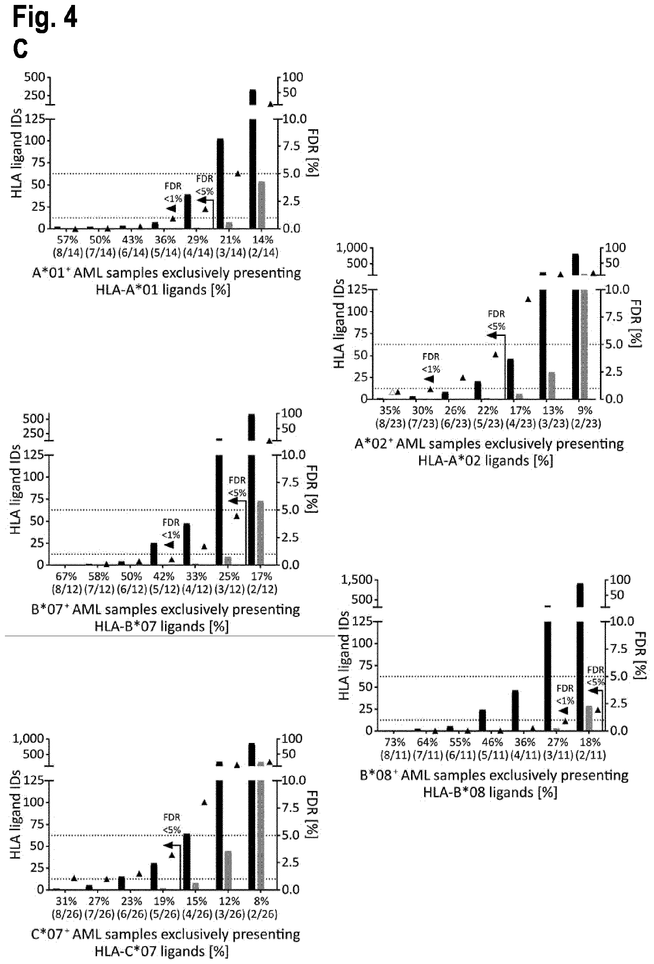

- Fig. 4:: HLA class I immunopeptidomics. (A) HLA-A (upper panel), HLA-B (middle panel), and HLA-C (lower panel) allotype frequencies in the AML (n = 47) and benign (n = 332) immunopeptidomics datasets. P values were determined by Chi-square test. (B) Allotype-specific comparative immunopeptidome profiling based on the frequency of HLA-restricted presentation in HLA-A*02 (upper left panel), HLA-B*07 (upper right panel), HLA-B*08 (lower left panel), and HLA-C*07 (lower right panel) positive AML and benign immunopeptidomes, respectively. Frequencies of positive immunopeptidomes (IPeps) for the respective HLA ligand (x-axis) are indicated on the y-axis. Comparative profiling was performed without one hit wonder peptides. (C) Statistical analysis of the proportion of false-positive AML-associated antigen identifications at different representation frequencies. The numbers of identified HLA-A*01-, HLA-A*02-, HLA-B*07-, HLA-B*08-, and HLA-C*07-restricted peptides based on immunopeptidome analysis of AML (n = 47) and benign (n = 332) cohorts were compared with random virtual (HLA-matched) AML-associated peptides (left y-axis), respectively. Virtual immunopeptidomes of AML and benign samples were generated *in silico* based on random weighted sampling from the entirety of peptide identifications in both original cohorts. These randomized virtual immunopeptidomes were used to define AML-associated antigens based on simulated cohorts of AML versus benign tissue samples. The process of peptide randomization, cohort assembly, and AML-associated antigen identification was repeated 1,000 times and the mean value of resultant virtual AML-associated antigens was calculated and plotted for the different threshold values. The corresponding false discovery rates (right y-axis) for any chosen threshold (x-axis) were calculated and the 1% and 5% false discovery rates are indicated within the plot (dotted lines and arrows). Abbreviations: IDs, identifications; FDR, false discovery rate.

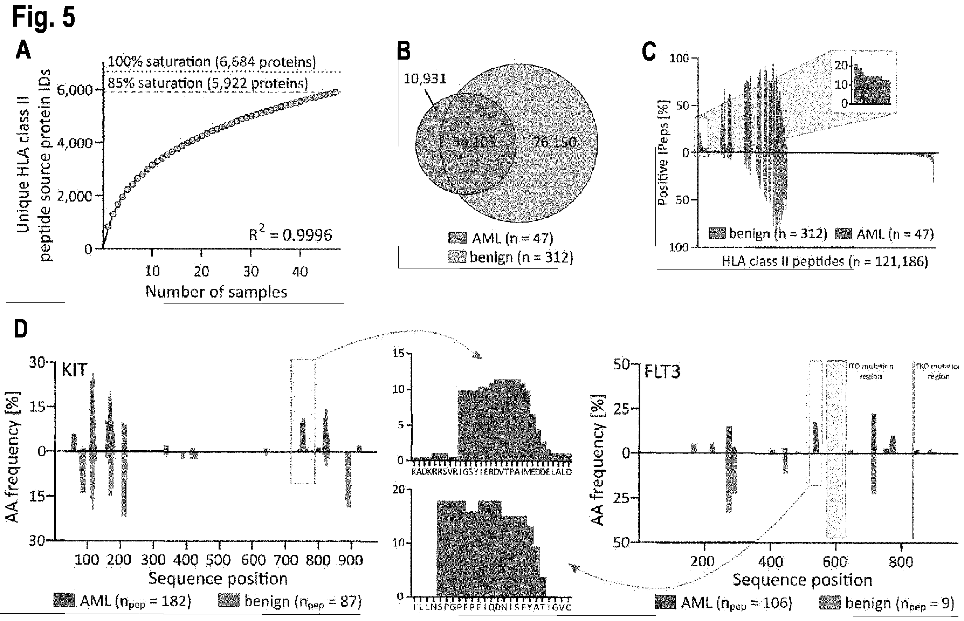

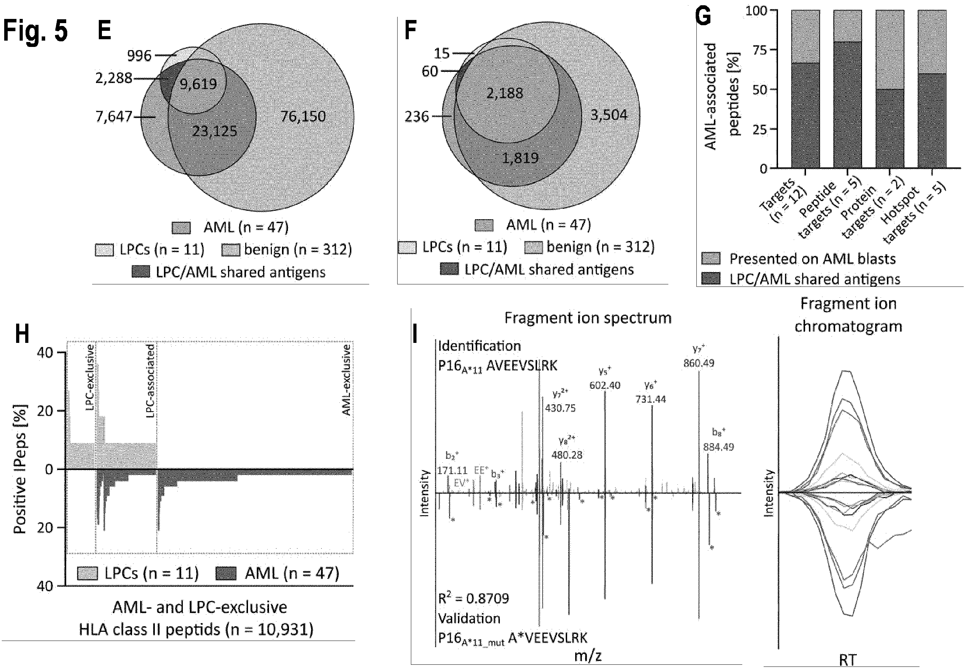

- Fig. 5:: Identification of AML- and LPC-associated HLA class II antigen targets by comparative immunopeptidome profiling. (A) Saturation analysis of HLA class II-restricted peptide source proteins of the AML cohort. Number of unique source protein identifications shown as a function of cumulative immunopeptidome analysis of AML samples (n = 47). Exponential regression allowed for the robust calculation of the maximum attainable number of different source protein identifications (dotted lines). The dashed line depicts the source immunoproteome coverage achieved in the AML cohort. (B) Overlap analysis of HLA class II peptide identifications of primary AML (n = 47) and benign samples (n = 312). (C) Comparative HLA class II immunopeptidome profiling based on the frequency of HLA-restricted presentation in AML and benign immunopeptidomes. Frequencies of positive immunopeptidomes for the respective HLA peptide (x-axis) are indicated on the y-axis. The box on the left and its magnification highlight the subset of AML-associated antigens showing AML-exclusive, high frequent presentation. (D) Hotspot analysis of the proteins KIT and FLT3 by HLA class II-presented peptide clustering, respectively. Representation frequencies of amino acid counts within each cohort for the respective amino acid position (x-axes) are indicated on the y-axes. The boxes and their magnifications highlight the identified hotspots with the respective amino acids on the x-axes. ITD and TKD mutation regions within FLT3 are marked with boxes. (E, F) Overlap analysis of HLA class II (E) peptide and (F) source protein identifications of LPCₑₙᵣ samples (n = 11) with AML (n = 47) and benign samples (n = 312). Overlap analysis, comparative profiling, and hotspot analysis were performed without one hit wonder peptides. (G) Analysis of the proportion of AML-associated HLA class II-presented peptide, protein, and hotspot targets that are presented on both AML blasts and LPCs (LPC/AML shared antigens). (H) Comparative profiling of AML- and LPC-exclusive HLA class II peptide identifications (not identified on benign tissue samples) based on the frequency of HLA-restricted presentation in the immunopeptidomes of LPC (n = 11) and AML (n = 47) samples. Frequencies of positive immunopeptidomes for the respective HLA peptide (x-axis) are indicated on the y-axis. The boxes mark subsets of LPC-exclusive antigens, LPC-associated antigens showing presentation on both LPCs and AML blasts, and AML-exclusive antigens. (I) Mass spectrometry-based neoantigen validation using an isotope-labeled synthetic peptide. The experimentally eluted peptide P16_{A*11_mut} (identification, above the x-axis) was validated with the isotope-labeled synthetic peptide (validation, mirrored on the x-axis). Identified b-, y- and internal ions are marked in the fragment ion spectrum (left site) in red, blue and orange, respectively. Ions containing the isotopic labeled amino acid alanine are marked with an asterisk. Coelution of the isotope-labeled synthetic peptide with the experimentally peptide validated equal retention times (fragment ion chromatogram, right panel). Abbreviations: AML, acute myeloid leukemia; IPep, immunopeptidome; n, number; AA, amino acid; nₚₑₚ, number of peptides; LPC, leukemic progenitor cells.

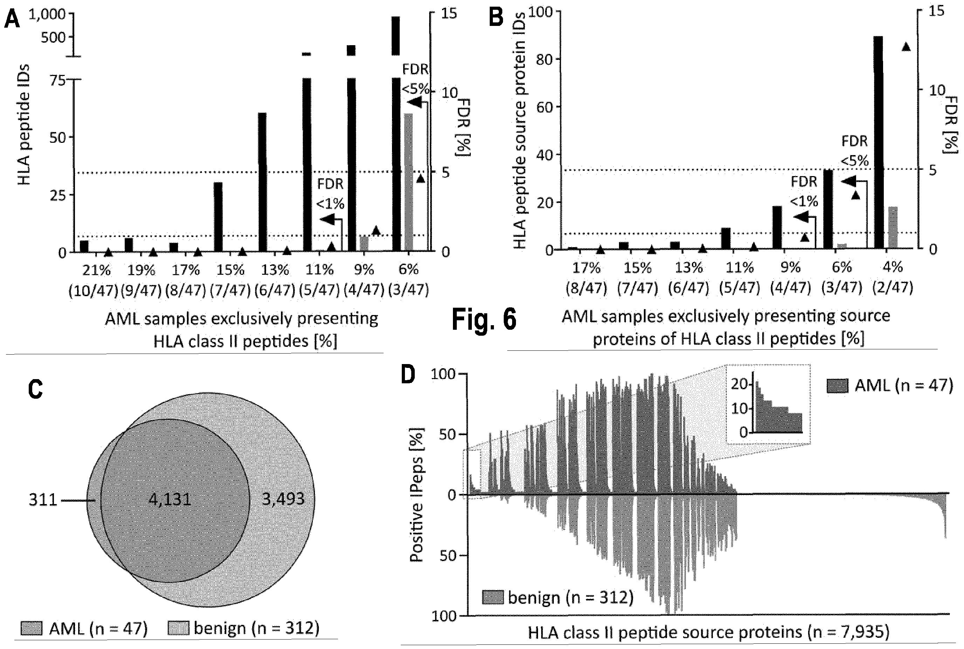

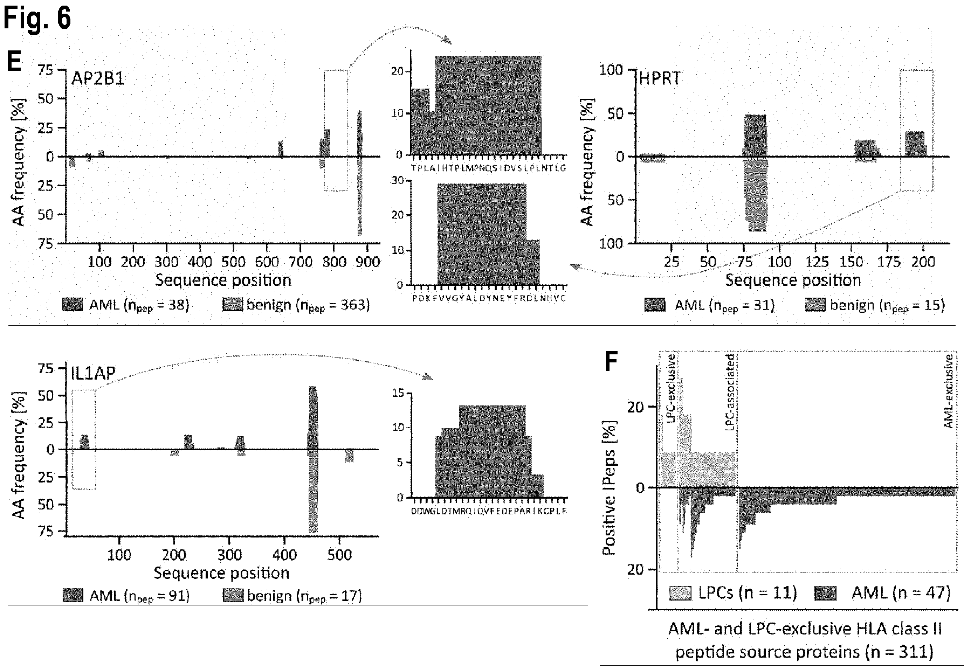

- Fig. 6:: Identification of AML- and LPC-associated HLA class II antigen targets by comparative immunopeptidome profiling. (A, B) Statistical analysis of the proportion of false-positive AML-associated antigen identifications at different representation frequencies. The numbers of identified HLA class II-restricted peptides (A) and peptide source proteins (B) based on immunopeptidome analysis of AML (n = 47) and benign (n = 332) cohorts were compared with random virtual AML-associated peptides and source proteins (left y-axis), respectively. Virtual immunopeptidomes of AML and benign samples were generated *in silico* based on random weighted sampling from the entirety of identifications in both original cohorts. These randomized virtual immunopeptidomes were used to define AML-associated antigens based on simulated cohorts of AML versus benign tissue samples. The process of peptide randomization, cohort assembly, and AML-associated antigen identification was repeated 1,000 times and the mean value of resultant virtual AML-associated antigens was calculated and plotted for the different threshold values. The corresponding false discovery rates (right y-axis) for any chosen threshold (x-axis) were calculated and the 1% and 5% false discovery rates are indicated within the plot (dotted lines and arrows). (C) Overlap analysis of HLA class II peptide source protein identifications of primary AML samples (n = 47) and benign samples (n = 312). (D) Comparative HLA class II immunopeptidome profiling on HLA peptide source protein level based on the frequency of HLA-restricted source protein presentation in AML and benign immunopeptidomes. Frequencies of positive immunopeptidomes for the respective HLA peptide source protein (x-axis) are indicated on the y-axis. The box on the left and its magnification highlight the subset of AML-associated antigens showing AML-exclusive, high frequent presentation. (E) Hotspot analysis of the proteins AP2B1, HPRT, and IL1AP by HLA class II-presented peptide clustering, respectively. Representation frequencies of amino acid counts within each cohort for the respective amino acid position (x-axes) are indicated on the y-axes. The boxes and their magnifications highlight the identified hotspots with the respective amino acids on the x-axes. (F) Comparative profiling of AML- and LPC-exclusive HLA class II source protein identifications (not identified on benign tissue samples) based on the frequency of HLA-restricted source protein presentation in the immunopeptidomes of LPC (n = 11) and AML (n = 47) samples. Frequencies of positive immunopeptidomes for the respective HLA peptide source protein (x-axis) are indicated on the y-axis. The boxes mark subsets of LPC-exclusive antigens, LPC-associated antigens showing presentation on both LPCs and AML blasts, and AML-exclusive antigens. Overlap analysis, comparative profiling, and hotspot analysis were performed without one hit wonder peptides. Abbreviations: AML, acute myeloid leukemia; IPep, immunopeptidome; n, number; AA, amino acid; nₚₑₚ, number of peptides; LPC, leukemic progenitor cells; IDs, identifications; FDR, false discovery rate.

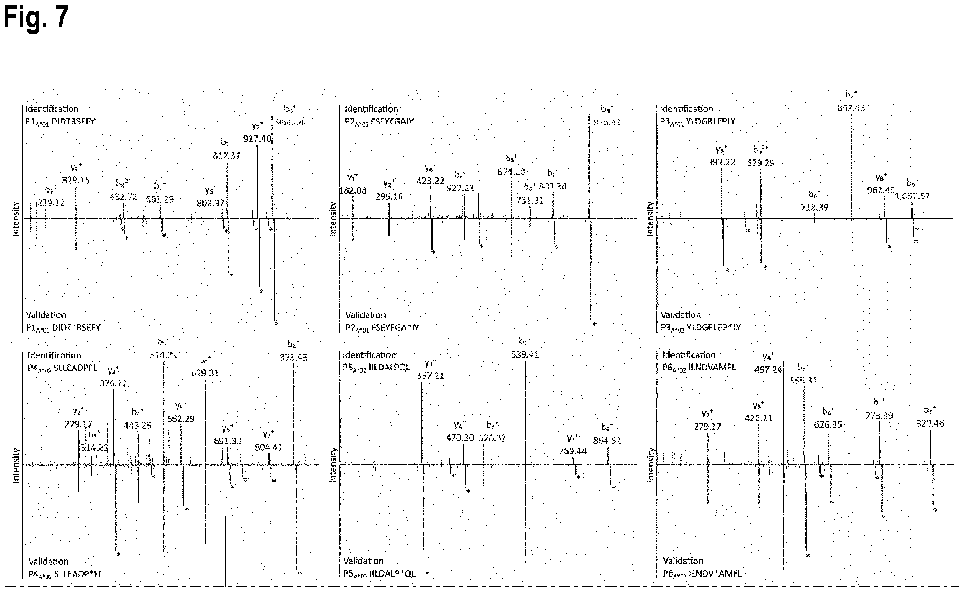

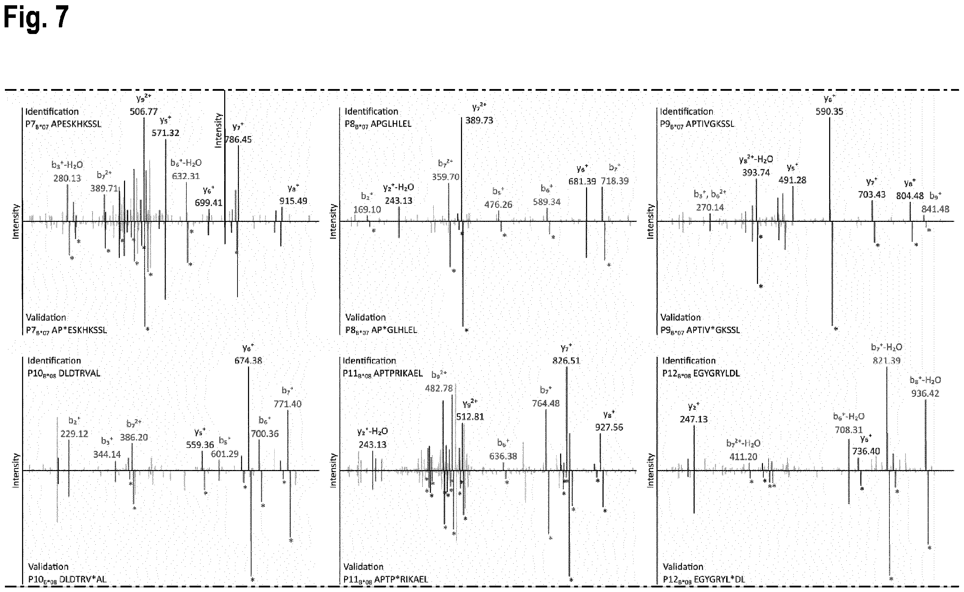

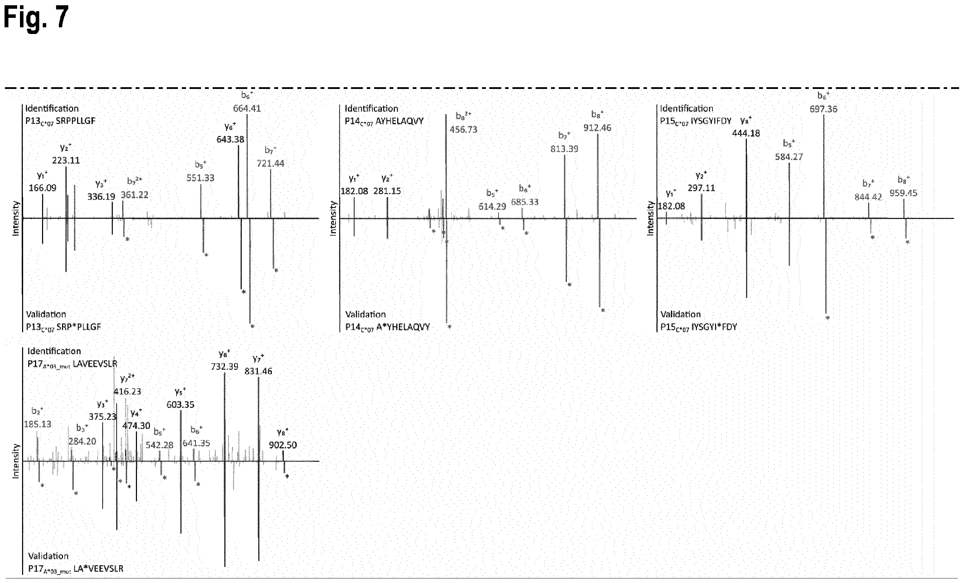

- Fig. 7:: Spectral validation of HLA class I-restricted AML- and LPC-associated peptides. Comparison of fragment spectra (m/z on x-axis) of HLA ligands eluted from primary samples (identification) to their corresponding isotope-labeled synthetic peptides (validation, mirrored on the x-axis). Identified b- and y-ions are marked in red and blue, respectively. Ions containing isotope-labeled amino acids are marked with asterisks.

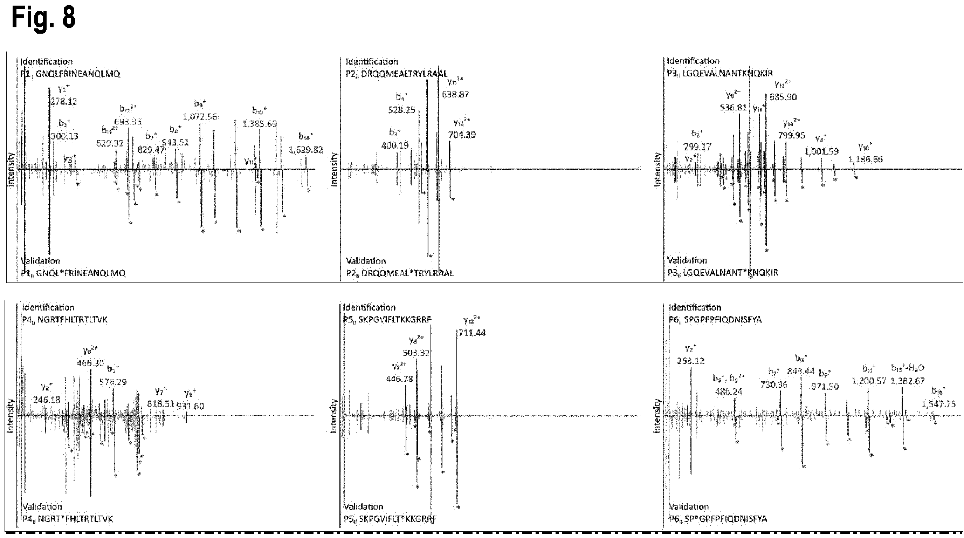

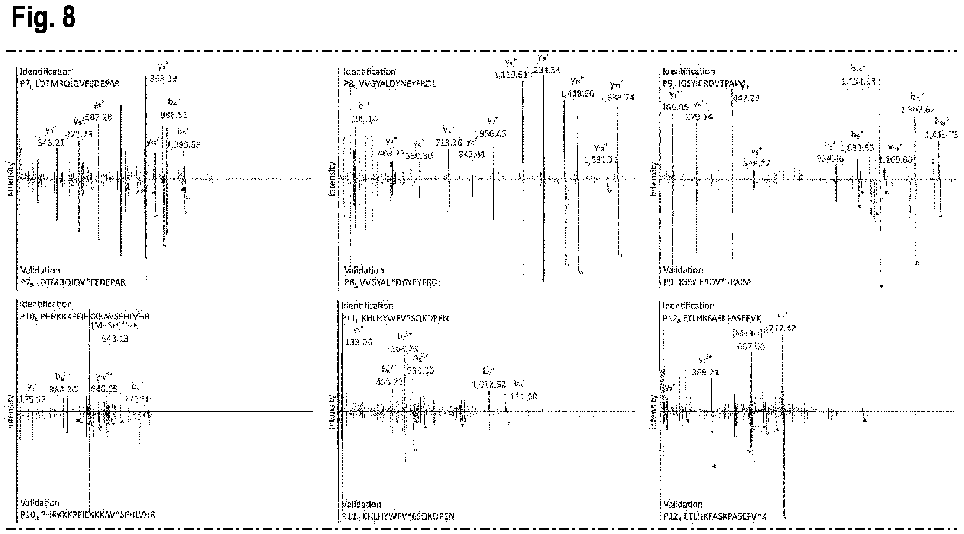

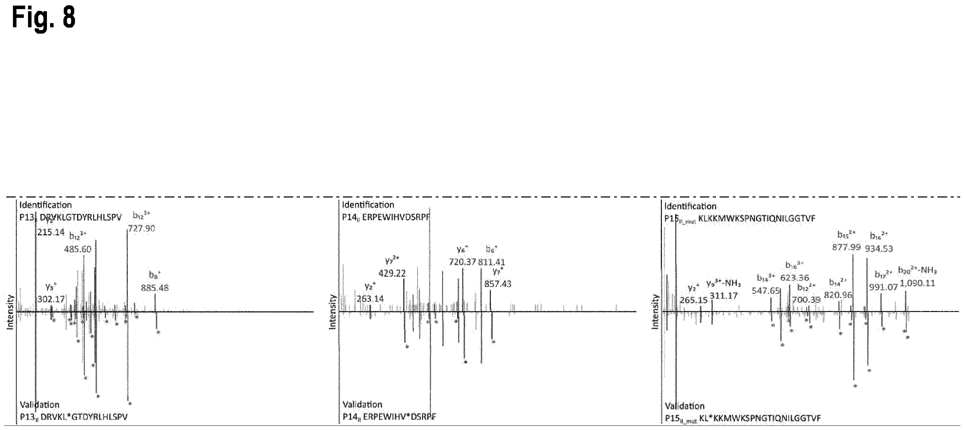

- Fig. 8:: Spectral validation of HLA class II-restricted AML- and LPC-associated peptides. Comparison of fragment spectra (*m*/*z* on x-axis) of HLA ligands eluted from primary samples (identification) to their corresponding isotope-labeled synthetic peptides (validation, mirrored on the x-axis). Identified b- and y-ions are marked in red and blue, respectively. Ions containing isotope-labeled amino acids are marked with asterisks.

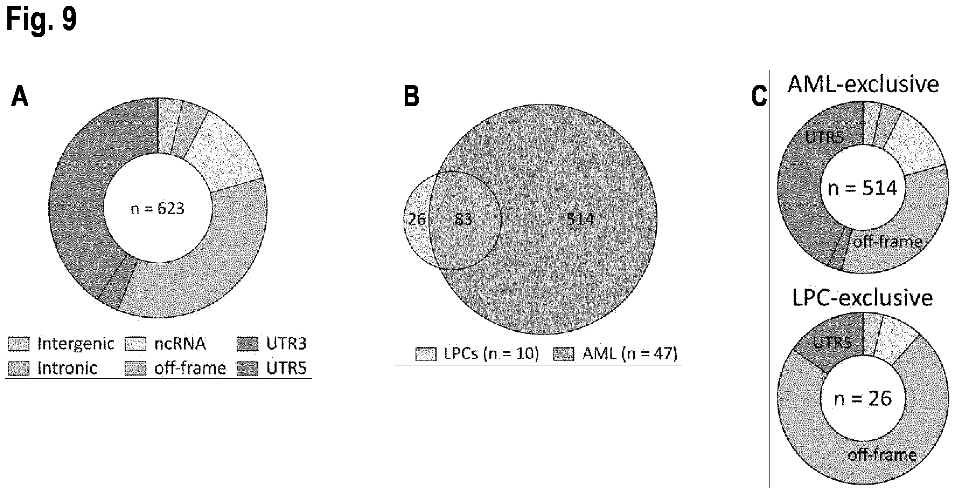

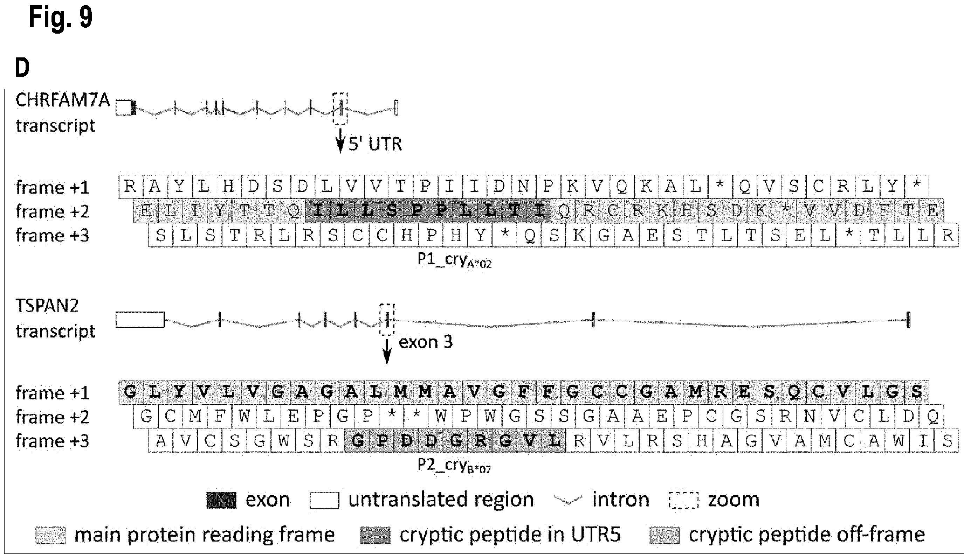

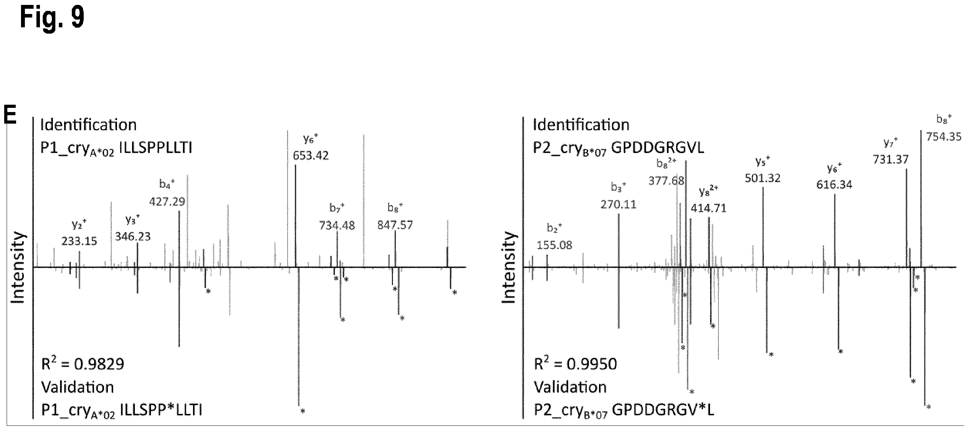

- Fig. 9:: Cryptic peptides are presented in AML- and LPC-derived immunopeptidomes. (A) Distribution of identified AML-associated cryptic peptides (n = 623) among genomic categories. AML-associated cryptic peptides were never identified on any benign tissue sample. (B) Overlap analysis of cryptic HLA class I ligand identifications of LPCₑₙᵣ samples (n = 10) with AML (n = 47). (C) Distribution of AML-exclusive (n = 514, upper panel) and LPC-exclusive (n = 26, lower panel) cryptic peptides among genomic categories. (D) Examples of a 5' UTR- (upper panel, P1_cry_{A*02}) and an off-frame-derived (lower panel, P2_cry_{B*07}) cryptic HLA peptides. The symbols depict the respective transcript of CHRFAM7A and TSPAN2, both on the reverse strand. The zoom box highlights the 5' UTR and exon 3 with the three reading frames including the cryptic peptides, respectively. (E) Spectral validation of the two cryptic peptides P1_cry_{A*02} (left panel) and P2_cry_{B*07} (right panel). Comparison of fragment spectra (*m*/*z* on x-axis) of cryptic peptides eluted from primary samples (identification) to their corresponding isotope-labeled synthetic peptides (validation, mirrored on the x-axis). Identified b- and y-ions are marked in red and blue, respectively. Ions containing isotope-labeled amino acids are marked with asterisks. Abbreviations: ncRNA, non-coding RNA; UTR, untranslated region; n, number; LPC, leukemic progenitor cells; AML, acute myeloid leukemia.

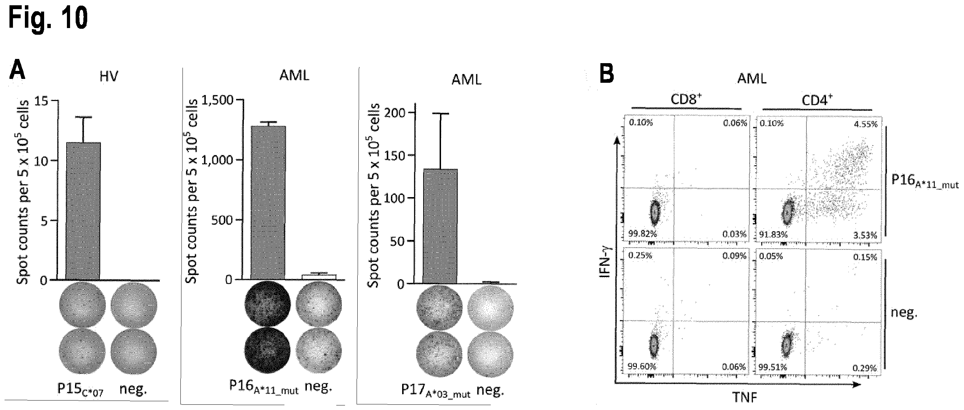

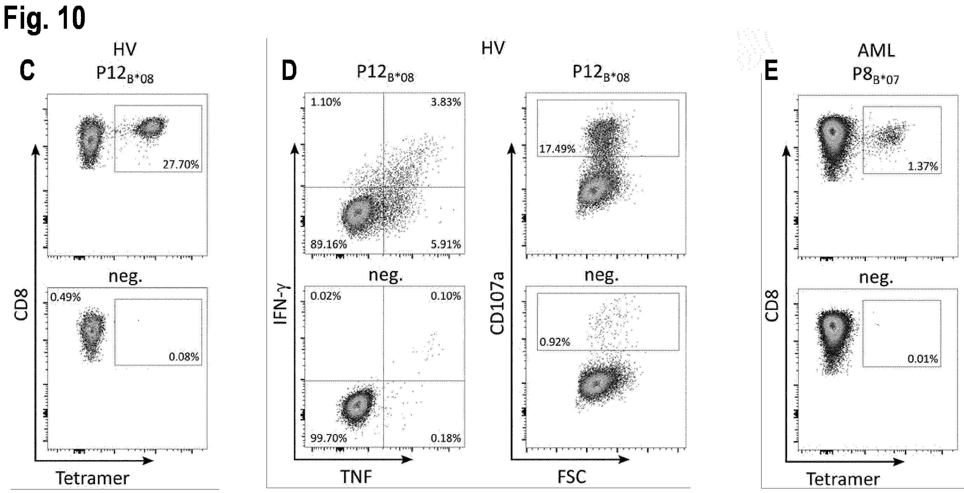

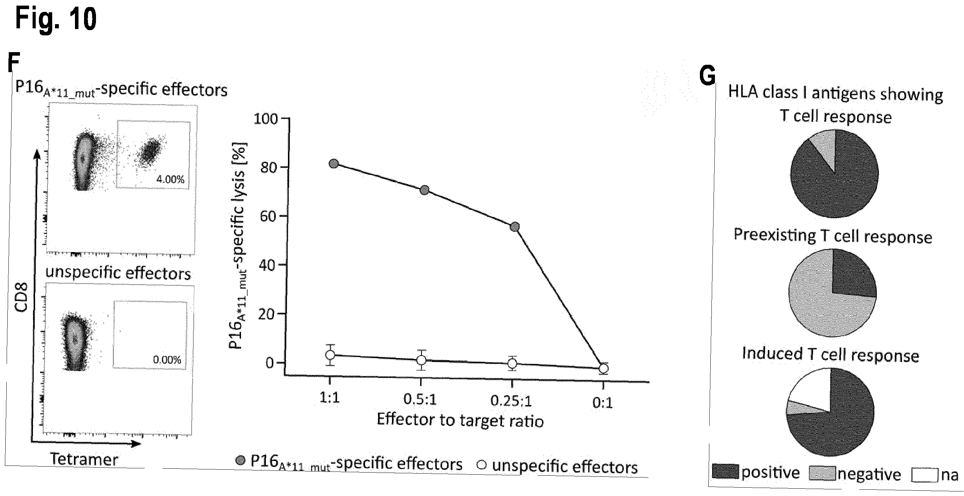

- Fig. 10:: Immunogenicity analysis of HLA class I-restricted targets. (A, B) Detection of preexisting peptide-specific T cell responses by (A) IFN-□ ELISpot assay and (B) intracellular cytokine staining after 12-day *in vitro* expansion using PBMC samples of HVs (A, left panel) and AML patients (A, middle and right panel, B). Representative examples are depicted. (A) Data are presented as bar graphs with mean and SD. (B) Graphs show single, viable cells stained for CD8 (left panel) and CD4 (right panel) and the cytokines IFN-γ and TNF. (C) *De novo* induction of peptide-specific CD8⁺ T cells using aAPC-based *in vitro* priming with PBMCs of an HV. Graphs show single, viable cells stained for CD8 and PE-conjugated multimers of indicated specificity. The negative control depicts antigen target tetramer staining of T cells from the same donor primed with an HLA-matched control peptide. (D) Functional characterization of induced P12_{B*08}-specific CD8⁺ T cells after in *vitro* aAPC-based priming by intracellular cytokine (IFN-γ, TNF) and degranulation marker (CD107a) staining. Representative example of IFN-□ and TNF production as well as CD107a expression after stimulation with the peptide P12_{B*08} (upper panel) compared to an HLA-matched control peptide (lower panel). Graphs show single, viable, CD8⁺ cells stained for IFN-γ and TNF (left panel) as well as CD107a (right panel). (E) *De novo* induction of peptide-specific CD8⁺ T cells with PBMCs of an AML patient. (F) Cytotoxicity of P16_{A*11_mut}-specific effector T cells analyzed in a VITAL cytotoxicity assay with *in vitro* primed CD8⁺ T cells from an HV. Tetramer staining of polyclonal effector cells before performance of the VITAL assay determined the amount of P16_{A*11_mut}-specific effector cells in the population of P16_{A*11_mut}-primed CD8⁺ T cells (upper panel) and in the population of control cells from the same donor primed with an HLA-matched control peptide (lower panel). Effector to target-dependent P16_{A*11_mut}-specific lysis of P16_{A*11_mut}-loaded autologous target cells in comparison to HLA-matched control peptide-loaded target cells (right panel). Unspecific effectors did not exhibit P16_{A*11_mut}-specific lysis of the same targets. Unspecific effectors were evaluated in three independent replicates and results are shown as mean with SD for the tree replicates. (G) Frequency of HLA class I-restricted antigenic peptides (n = 17) that elicited detectable preexisting T cell responses in IFN-γ ELISpot assays (upper panel), *de novo* inducible T cell responses after aAPC-based *in vitro* priming (middle panel), or showed any type of immunogenicity (lower panel) depicted as pie charts. Abbreviations: neg., HLA-matched negative control peptide; FSC, forward scatter; na, not available.

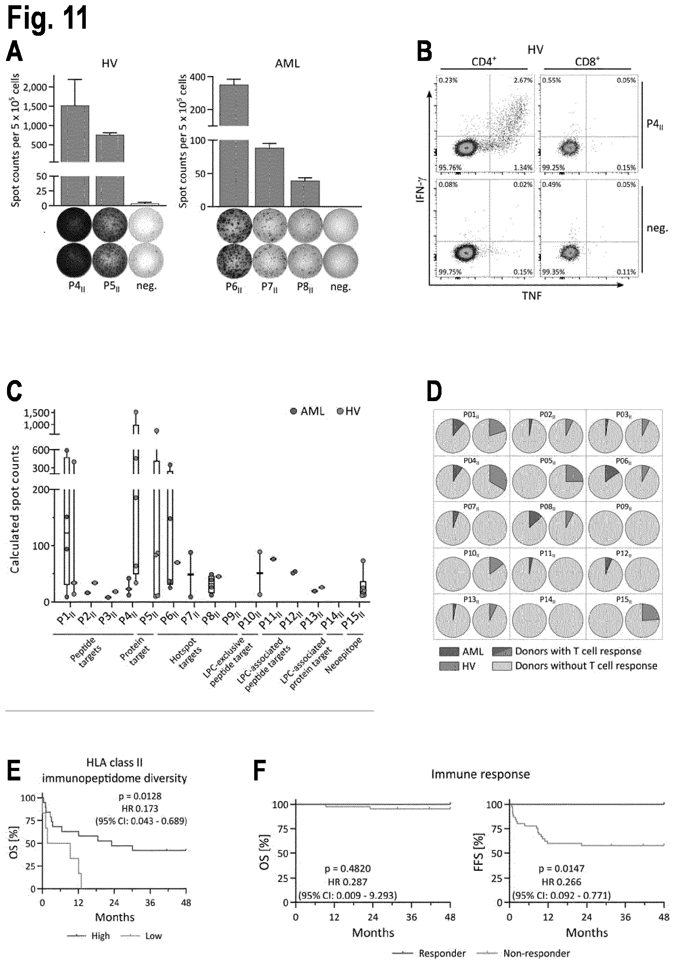

- Fig. 11:: Immunogenicity analysis of HLA class II-restricted targets and impact of immunopeptidome diversity and peptide-specific immune responses on patient survival. (A, B) Detection of preexisting peptide-specific T cell responses by (A) IFN-γ ELISpot assay and (B) intracellular cytokine staining after 12-day *in vitro* expansion using PBMC samples of HVs (A, left panel, B) and AML patients (A, right panel). Representative examples are depicted. (A) Data are presented as bar graphs with mean and SD. (B) Graphs show single, viable cells stained for CD4 (left panel) and CD8 (right panel) and the cytokines IFN-γ and TNF. (C) Intensity of T cell responses in terms of calculated spot counts in IFN-□ ELISPOT assays after 12-day stimulation against the respective AML- and LPC-associated HLA class II-restricted antigenic peptide using PBMCs of AML patients and HVs. Dots represent data from individual donors. Boxes represent median and 25^{th} to 75^{th} percentiles, whiskers are minimum to maximum. (D) Pie charts depicting the recognition frequency (individuals with T cell responses/tested individuals) of AML- and LPC-associated HLA class II-restricted antigen peptides in PBMC samples of AML patients and HVs as assessed by IFN-γ ELISpot assay after 12-day stimulation. Up to 37 AML patient samples and 25 HV samples were tested per peptide. (E) Impact of HLA class II-restricted immunopeptidome diversity in terms of unique AML-exclusive HLA class II-restricted peptide presentation on overall survival of AML patients (n = 25). (F) Impact of antigen-specific immune responses against HLA class II-restricted AML- and LPC-associated peptides on overall (OS, left panel) and treatment failure-free (FFS, right panel) survival of AML patients (n = 56). Kaplan-Meier analysis, log-rank test. Abbreviations: Cl, confidence interval; HR, hazard ratio; HV, healthy volunteer; neg., negative peptide; AML, acute myeloid leukemia; LPC, leukemic progenitor cells.

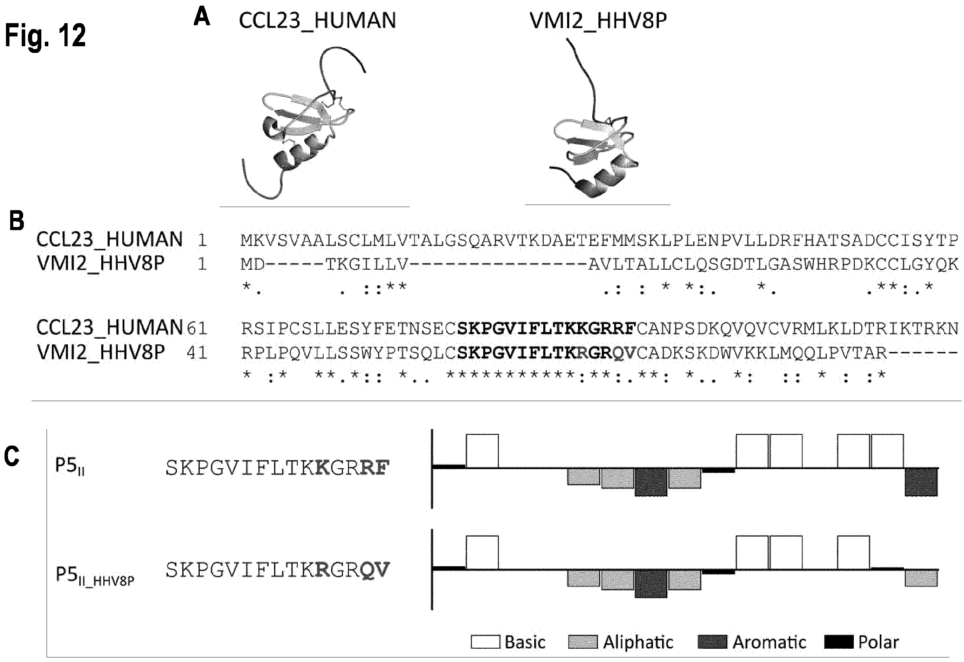

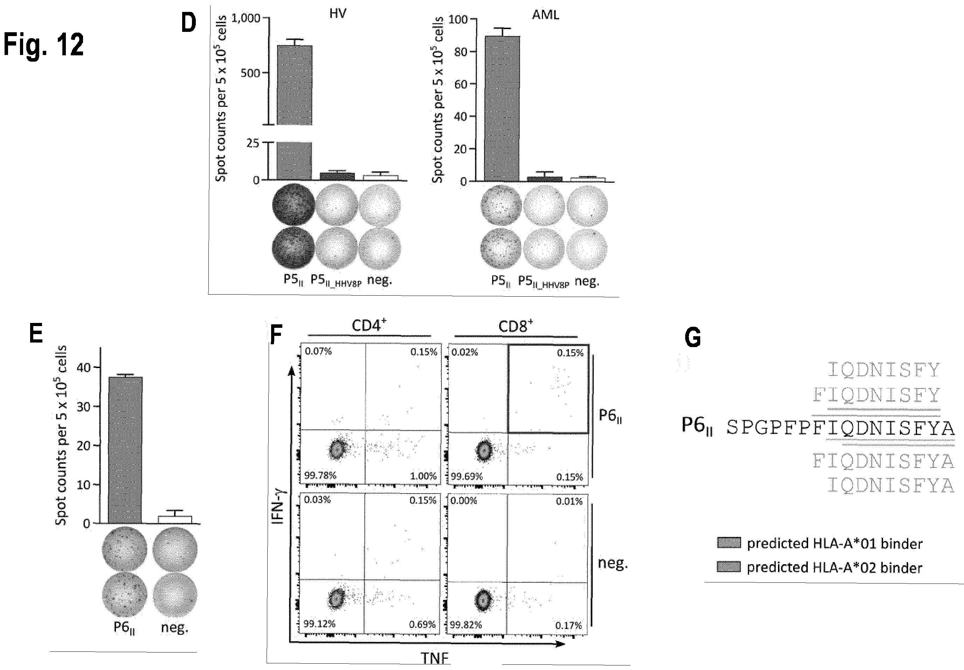

- Fig. 12:: Further characterization of HLA class II-restricted antigens. (A) Structural comparison of CCL23_HUMAN the source protein of the AML-associated target P5II with the viral protein VMI2_HHV8P of the human herpes virus 8 (HHV8). (B) Sequence alignment of CCL23_HUMAN and VMI2_HHV8P with the HLA-presented peptide P5II and its viral counterpart peptide highlighted in bold. Non-similar amino acids are marked in red. The symbols underneath denoting the degree of conservation. An asterisk indicates fully similar amino acids, a colon and a period indicate amino acids with strongly and weakly similar properties, respectively. (C) Physiochemical property alignments of P5_{II} and its viral counterpart P5_{II_HHV8P}. Physiochemical properties were calculated by the PepCalc software. Column directions (up versus down) indicate hydrophilicity according to the Hopp-Woods scale. (D) Detection of preexisting P5_{II}-specific T cell responses but not of against the viral counterpart P5_{II_-HHV8P} by IFN-γ ELISpot assay after 12-day *in vitro* expansion using PBMC samples of HVs. Representative examples are depicted. Data are presented as bar graphs with mean and SD. (E) P6_{II}-specific T cell responses as assessed by IFN-γ ELISpot assay after 12-day stimulation using PBMC samples of an AML patient. (F) Functional characterization of P6_{II}-specific T cells by flow cytometry-based intracellular cytokine staining revealed a CD8⁺ T cell-driven P6_{II}-specific response. (G) *In silico* peptide prediction revealed four possible embedded HLA class I-restricted peptides for the HLA allotypes of the patient (A*01, A*02, B*08, B*13, C*06, C*07). Abbreviations: neg., negative.

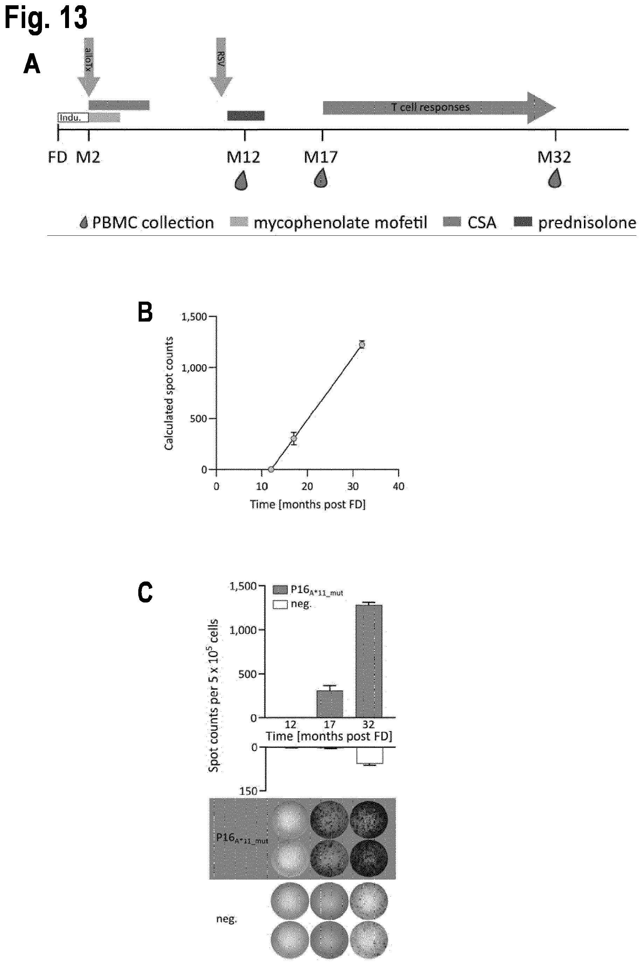

- Fig. 13:: Longitudinal analysis of peptide-specific T cell responses after allogenic stem cell transplantation. (A) Schematic therapy course of the AML patient UPN23. After first diagnosis (FD) the patient received induction therapy (idarubicin/cytarabine) followed by allogenic stem cell transplantation (alloTx) two months after FD. The patient received different immunosuppressive therapies (mycophenolate mofetil, cyclosporine (CSA), prednisolone) after alloTx and a respiratory syncytial virus (RSV) pneumonia. At month 12, 17 and 32 after FD AML-associated peptide-specific T cell responses were assessed by IFN-γ ELISpot assay. (B, C) P16_{A*11_mut}-specific T cell responses at different time points after allogenic stem cell transplantation in UPN23. Calculated spot counts depict the mean spot count of duplicates normalized to 5 × 10⁵ cells minus the normalized mean spot count of the respective negative control.

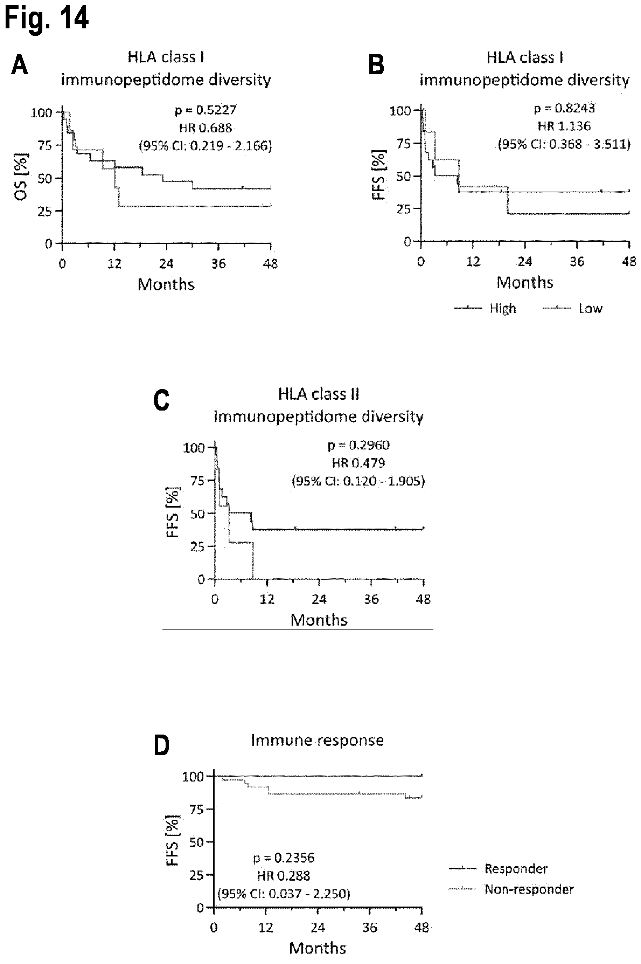

- Fig. 14:: Impact of impact of immunopeptidome diversity and peptide-specific immune responses on patient survival. (A-C) Impact of HLA class I- (A, B) and HLA class II-restricted (C) immunopeptidome diversity in terms of unique AML-exclusive peptide presentation on overall (OS, A) and treatment failure-free (FFS, B, C) survival of AML patients (n = 26 for HLA class I, n = 25 for HLA class II). (D) Impact of antigen-specific immune responses against HLA class II-restricted AML- and LPC-associated peptides on treatment FFS of AML patients (n = 45) after allogenic stem cell transplantation. Kaplan-Meier analysis, log-rank test. Abbreviations: HR, hazard ratio; Cl, confidence interval.

### 1. Material and Methods

### Patients and blood samples

For immunopeptidome analysis, PBMCs or bone marrow mononuclear cells (BMNCs) from AML patients at the time of diagnosis, at relapse, or in MR were collected at the Departments of Hematology and Oncology in Tübingen and Dresden, Germany, as well as at the Department of Medicine, Divisions of Hematology and Medical Oncology at the San Francisco University of California, CA, United States of America. For T cell-based assays, PBMCs from HVs and AML patients after allogenic stem cell transplantation or in complete remission at different time points were collected. Cells were isolated by density gradient centrifugation and stored at -80°C. Clinical and survival data were collected with a follow-up phase of up to 48 months after date of diagnosis. Informed consent was obtained in accordance with the Declaration of Helsinki protocol. The study was performed according to the guidelines of the local ethics committees. HLA typing was carried out by the Department of Hematology and Oncology, Tübingen, Germany.

### HLA surface quantification

HLA surface expression was determined using the QIFIKIT bead-based quantification flow cytometric assay (Dako) according to the manufacturer's instructions. In brief, cells were stained either with the pan-HLA class I-specific W6/32 monoclonal antibody, the HLA-DR-specific L243 monoclonal antibody (produced in-house), or IgG isotype control (BioLegend), respectively. Polyclonal goat FITC anti-mouse antibody (Dako) was used as secondary antibody. After washing with normal mouse serum (affymetrix eBioscience) surface marker staining was performed using PE/Cy7 anti-human CD38 (BioLegend), APC anti-human CD34 (BD), and Pacific Blue anti-human CD45 antibodies (BD). Aqua fluorescent reactive dye (Invitrogen) was used as viability marker. Analyses were performed on a FACS Canto II cytometer (BD). Only cell populations with ≥ 100 cells were analyzed for their HLA surface expression.

### LPC enrichment

Enrichment of LPCs from AML samples were either performed by fluorescence-activated cell sorting (FACS) at the Institute for Stem Cell Biology and Regenerative Medicine, Stanford, CA, United States (UPN3-8, UPN11) or by magnetic-activated cell sorting (MACS) at the Institute for Cell Biology, Department of Immunology, University of Tübingen, Tübingen, Germany (UPN01, UPN02, UPN09, UPN10). For FACS, PBMCs were stained with PE/Cy7 anti-human CD38, APC anti-human CD34, and PerCP/Cy5.5 anti-human CD3, CD19, CD20, and CD56 monoclonal antibodies and sorted on a Beckton Dickinson FACSAria II or FACSAria III. MACS was performed with the human CD34 Multi-Sort and CD38 MicroBead Kits (both Miltenyi). Sorted cells were stained with PE/Cy7 anti-human CD38 (BioLegend), APC anti-human CD34 (BD), and Pacific Blue anti-human CD45 (BD) monoclonal antibodies to determine the purity. Aqua fluorescent reactive dye (Invitrogen) was used as viability marker. Analyses were performed on a FACS Canto II cytometer (BD).

CD34⁺ HPCs were magnetically enriched (CD34 MicroBead Kit, human, Miltenyi) from hematopoietic stem cell aphereses from G-CSF mobilized blood donations of HVs and patients with non-hematological malignancies (e.g., germ cell tumors).

### Mice and xenotransplantation assays

Xenotransplantation assays were performed at the Department of Bio-medicine, University of Basel and University Hospital Basel, Switzerland. NOD.Cg-*Prkdc^{Scid} IL2rg^{tmWjl}*/*Sz* mice (NSG, Jackson Laboratory) were maintained under pathogen-free conditions according to the Swiss federal and state regulations. All animal experiments were approved by the Veterin6ramt Basel-Stadt. Xenotransplantation assays were performed as previously described. In brief, 6 × 10⁵ primary human presorted CD34⁺CD38⁻ and CD34⁺CD38⁺ AML cells were transplanted via intrafemoral injection into eight weeks old female NSG mice (n = 4 - 5 per group). Engraftment was monitored as previously described via routine bone marrow punctures or assessment of peripheral blood. Engraftment was defined as ≥ 1% human leukemic cells in murine PB or BM as analyzed by multicolor flow cytometry using antibodies against human leukemic antigens. The panel includes fluorescent antibodies against human CD33, CD34, CD133, CD117, CD45 (BD Biosciences), CD14, CD13 (eBiosciences), CD3, and CD19 (BioLegend). All mice underwent final BM, PB and organ assessment by multicolor flow cytometry.

### Isolation of HLA ligands

HLA class I and HLA class II molecules were isolated by standard immunoaffinity purification32 using the pan-HLA class I-specific W6/32, the pan-HLA class II-specific Tü-39, and the HLA-DR-specific L243 monoclonal antibodies (all produced in-house, University of Tübingen, Department of Immunology) to extract HLA ligands.

### Mass spectrometric data acquisition

The mass spectrometric analysis was performed as described previously. Peptides were separated by nanoflow high-performance liquid chromatography (RSLCnano, Thermo Fisher) using a 50 µm × 25 cm PepMap rapid separation column (Thermo Fisher) and a gradient ranging from 2.4 to 32.0% acetonitrile over the course of 90 min. Eluting peptides were analyzed in an online-coupled Orbitrap Fusion Lumos mass spectrometer (Thermo Fisher) equipped with a nanoelectron spray ion source using a data dependent acquisition mode employing a top speed collisional-induced dissociation (CID, HLA class I peptides) or higher-energy collisional dissociation (HCD, HLA class II peptides) fragmentation method (normalized collision energy 35%). Mass range for HLA class I peptide analysis was set to 400 - 650 m/z with charge states 2+ and 3+ selected for fragmentation. For HLA class II peptide analysis mass range was limited to 400 - 1,000 m/z with charge states 2+ to 5+ selected for fragmentation.

### Data processing

Data processing was performed as described previously. In brief, the SEQUEST HT search engine (University of Washington) was used to search the human proteome as comprised in the Swiss-Prot database (20,279 reviewed protein sequences, September 27th 2013) without enzymatic restriction. Precursor mass tolerance was set to 5 ppm, and fragment mass tolerance to 0.02 Da. Oxidized methionine was allowed as a dynamic modification. The FDR was estimated using the Percolator algorithm and limited to 5% for HLA class I and 1% for HLA class II. Peptide lengths were limited to 8 - 12 amino acids for HLA class I and to 8 - 25 amino acids for HLA class II. Protein inference was disabled, allowing for multiple protein annotations of peptides. HLA class I annotation was performed using NetMHCpan 4.0 and SYFPEITHI annotating peptides with percentile rank below 2% and ≥ 60% of the maximal score, respectively. For comparative profiling peptides only presented on one sample with a PSM count ≤ 3 ("one hit wonders") were removed.

### Screening for neoepitopes

For neoepitope screening, we used a non-patient-individual mutFASTA, which includes the TOP100 recurrent AML-associated missense mutations specified in the COSMIC database (www.cancer.sanger.ac.uk; Forbes, S. A. et al. COSMIC: somatic cancer genetics at high-resolution. Nucleic Acids Res 45, D777-D783 (2017)) supplemented with the most common NPM1 frame shift mutations (type A, B, C, D, and E; Falini, B. et al. Cytoplasmic nucleophosmin in acute myelogenous leukemia with a normal karyo-type. N Engl J Med 352, 254-266 (2005)) as well as FLT3-ITD (Smith, C. C. et al. Validation of ITD mutations in FLT3 as a therapeutic target in human acute myeloid leukaemia. Nature 485, 260-263 (2012)) and FLT3-TKD mutations (Opatz, S. et al. Exome sequencing identifies recurring FLT3 N676K mutations in core-binding factor leukemia. Blood 122, 1761-1769 (2013); Bacher et al. Prognostic relevance of FLT3-TKD mutations in AML: the combination matters--an analysis of 3082 patients. Blood 111, 2527-2537 (2008); Thiede, C. et al. Analysis of FLT3-activating mutations in 979 patients with acute myelogenous leukemia: association with FAB subtypes and identification of subgroups with poor prognosis. Blood 99, 4326-4335 (2002); Vempati, S. et al. Arginine 595 is duplicated in patients with acute leukemias carrying internal tandem duplications of FLT3 and modulates its transforming potential. Blood 110, 686-694 (2007); Yamamoto, Y. et al. Activating mutation of D835 within the activation loop of FLT3 in human hematologic malignancies. Blood 97, 2434-2439 (2001)). Data processing of AML immunopeptidomics data with the mutFASTA were performed as described above. To minimize false positive identifications, more stringent filter criteria with 5% FDR for HLA class I and 1% for HLA class II, XCorr ≥ 1, and ΔScore ≥ 0.2 were applied. After manual spectrum validation, candidate neoepitopes were produced as isotope-labeled synthetic peptides and used for spectral comparison and validation.

### Peptide synthesis and spectrum validation

Peptides were produced by the peptide synthesizer Liberty Blue (CEM) using the 9 fluorenylmethyl-oxycarbonyl/tert-butyl strategy. Spectrum validation of the experimentally eluted peptides was performed by computing the similarity of the spectra with corresponding isotope-labeled synthetic peptides measured in a complex matrix. Fragments of the synthetic peptides which contain an isotope label have a mass shift compared to non-labeled fragments and therefore penalize spectral similarity score. To minimize such effects, the spectra of synthetic peptides were preprocessed by shifting isotope label containing single- or double-charged b- and y-ion peaks to the m/z position where their corresponding unlabeled fragment is supposed to be. The spectral correlation was then calculated between eluted peptide spectra and preprocessed synthetic peptide spectra using the intensities of annotated b- and y-ion peaks.

### Validation of mutation-derived HLA ligands by targeted PRM

Validation of the NPM1 mutation-derived HLA-A*11-restricted peptide AVEEVSLRK (P16_{A*11_MUT}) was performed using the sensitive targeted PRM MS method. To generate the PRM assay library, the synthetic heavy isotope-labeled reference peptide was first spiked-in a complex biological matrix (HLA ligands isolated from JY cells) and analyzed on an LTQ Orbitrap Fusion Lumos mass spectrometer in PRM-triggered MS2 mode. For the validation of the mutation-derived HLA ligand the reference peptide was spiked-in the respective samples and analyzed with a PRM MS method. The mass spectrometer was operated in PRM mode, triggering acquisition of a full mass spectrum at 120,000 resolution (AGC target 1.5 × 10⁵, 50 ms maximum injection time) in the orbitrap followed by isolation of precursor ions in the quadrupole and MS2 scans in the orbitrap at 60,000 resolution (AGC target 7.0 × 10⁴, 118 ms maximum injection time) as triggered by a scheduled inclusion list containing the masses of interest of the mutation-derived peptide with charge states 2+ and 3+ as well as the respective masses of the heavy isotope-labeled reference peptide. Ion activation was performed by CID at normalized collision energy of 35%. The software Skyline (version 3.7.0) was used to generate a spectral library from the msf files. The transition settings were set to precursor charges 2 and 3, ion charges *1* and *2,* ion types *y, b, a*, and *p,* product ions from *ion 2* to *last ion -2.* The ion match tolerance for the library was set to *0.02 m*/*z* and 8 product ions are picked. The method match tolerance was set to *0.02 m*/*z* as well as the acquisition method for the MS/MS filtering to *targeted.*

### Identification of cryptic peptides

Cryptic HLA class I peptides were identified using Peptide-PRISM as described recently. *De novo* peptide sequencing was performed with PEAKS X (Bioinformatics Solutions Inc, Canada). Raw data refinement was performed with the following settings: (1) merge options: no merge; (2) precursor options: corrected; (iii) charge options: no correction; (4) filter options: no filter; (5) process: true; (6) default: true; and (7) associate chimera: yes. *De novo* sequencing was performed with parent mass error tolerance set to 10 ppm. Fragment mass error tolerance was set to 0.15 Da, and enzyme was set to none. The following variable modifications have been used: oxidation (M), pyro-Glu from Q (N-term Q), and carbamidomethylation (C). A maximum of three variable post-translational modifications were allowed per peptide. Up to 10 *de novo* sequencing candidates were reported for each identified fragment ion mass spectrum, with their corresponding average local confidence score. Because we applied the chimeric spectra option of PEAKS X, two or more TOP10 candidate lists could be assigned to a single fragment ion spectrum. Two tables ('all *de novo* candidates' and *'de novo* peptides') were exported from PEAKS for further analysis.

All de novo sequence candidates were matched against the six-frame translated human genome (hg38) and the three-frame translated human transcriptome (ENSEMBL 90) using Peptide-PRISM. Results were filtered to 10% FDR for each category (CDS, UTR5, OffFrame, ncRNA, UTR3, intronic, and intergenic). NetMHCpan 4.0 was used to predict binding affinities for all identified HLA class I peptides for all HLA alleles of the corresponding sample.

### Amplification of peptide-specific T cells and IFN-γ ELISpot assay

PBMCs from AML patients and HVs were pulsed with 1 µg/mL (HLA class I) or 5 µg/mL (HLA class II) per peptide and cultured for 12 days adding 20 U/mL IL-2 (Novartis) on days 3, 5, and 7. Peptide-stimulated PBMCs were analyzed by ELISpot assay on day 12. Spots were counted using an ImmunoSpot S5 analyzer (CTL) and T cell responses were considered positive when > 10 spots/500,000 cells were counted and the mean spot count was at least three-fold higher than the mean spot count of the negative control.

### Refolding

Biotinylated HLA-peptide complexes were manufactured as described previously and tetramerized using PE-conjugated streptavidin (Invitrogen Life Technologies) at a 4:1 molar ratio.

### Induction of peptide-specific CD8⁺ T cells with aAPCs

Priming of peptide-specific CTLs was conducted using aAPCs as described before. In detail, 800 000 streptavidin-coated microspheres (5.6 µm diameter, Bangs Laboratories) were loaded with 200 ng biotinylated peptide-HLA complexes and 600 ng biotinylated anti-human CD28 antibody (clone 9.3, in-house production). MACS-sorted CD8⁺ T cells (CD8 Microbeads, human, Miltenyi) were cultured with 4.8 U/µl IL 2 (R+D) and 1.25 ng/ml IL 7 (PromoKine). Weekly stimulation with aAPCs (200,000 aAPCs per 1 × 10⁶ CD8⁺ T cells) and 5 ng/ml IL-12 (PromoKine) was performed four times. Induction of peptide-specific T cells was analyzed by tetramer staining.

### Cytokine and tetramer staining

The frequency and functionality of peptide-specific CD8⁺ T cells was analyzed by tetramer and ICS as described previously. For ICS, cells were pulsed with 10 µg/mL of individual peptide and incubated with 10 µg/mL Brefeldin A (Sigma-Aldrich) and 10 µg/mL GolgiStop (BD) for 12 - 16 h. Staining was performed using Cytofix/Cytoperm (BD), PerCP anti-human CD8, Pacific Blue anti-human TNF, FITC anti-human CD107a (BioLegend), and PE anti-human IFN-γ monoclonal antibodies (BD). PMA and ionomycin (Sigma-Aldrich) served as positive control. The following peptides were used as negative control peptides: GSEELRSLY (SEQ ID NO: 33, POL_HV1BR, HLA-A*01; YLLPAIVHI (SEQ ID NO: 34), DDX5_HUMAN, HLA-A*02; RLRPGGKKK (SEQ ID NO: 35), GAG_HV1BR, HLA-A*03; TPGPGVRYPL (SEQ ID NO: 36), NEF_HV1BR, HLA-B*07; DIAARNVL (SEQ ID NO: 37), FAK1_HUMAN, HLA-B*08; ASEDYVAPPK (SEQ ID NO: 38), MKX_HUMAN, HLA-A*11; ETVITVDTKAAGKGK (SEQ ID NO: 39), FLNA_HUMAN, HLA class II. The frequency of peptide-specific CD8⁺ T cells after aAPC-based priming was determined by PE/Cy7 anti-human CD8 monoclonal antibody (Biolegend) and HLA:peptide tetramer-PE staining. Tetramers of the same HLA allotype containing irrelevant control peptides were used as negative control. The priming was considered successful if the frequency of peptide-specific CD8⁺ T cells was > 0.1% of CD8⁺ T cells within the viable single cell population and at least three-fold higher than the frequency of peptide-specific CD8⁺ T cells in the negative control. The same evaluation criteria were applied for ICS results. Samples were analyzed on a FACS Canto II cytometer (BD).

### Cytotoxicity assay

Cytolytic capacity of peptide-specific CD8⁺ T cells was analyzed using the flow cytometry-based VITAL assay as described before. Autologous CD8-depleted PBMCs were loaded with the test peptide or an HLA-matched control peptide and labeled with CFSE or FarRed, respectively. Effector cells were added in the indicated effector to target ratios. Specific lysis of peptide-loaded target cells was calculated relative to control targets.

### Quantification and statistical analysis

Overlap analysis was performed using BioVenn. The population coverage of HLA allotypes was calculated by the IEDB population coverage tool (www.iedb.org). An in-house Python script was used for the calculation of FDRs of AML-associated peptides at different presentation frequencies. Hotspot analysis (hotspot length ≥ 8 amino acids) of HLA class II immunopeptidomes was performed using an in-house R script that maps identified peptides according to their sequence onto its source protein and calculates representation frequencies of single amino acid positions within the respective cohorts. Flow cytometric data was analyzed using FlowJo 10.0.8 (Treestar). For survival analysis investigating the impact of the immunopeptidome diversity, peptide yields of AML-exclusive peptides were normalized to the cell number applied for immunopeptidome analysis. OS and FFS were depicted for low and high immunopeptidome diversity according to the median peptide yields and calculated by Kaplan-Meier method. The log-rank test was performed to test the difference of survival between the groups. For survival analysis investigating the impact of pre-existing antigen-specific immune responses against HLA class II-restricted AML- and LPC-associated peptides as detected by IFN-γ ELISpot assays patients were dichotomized into the group of responders showing a peptide-specific T cell response and non-responders without any detectable peptide-specific T cell response. All figures and statistical analyses were generated using GraphPad Prism 9.4.1 (GraphPad Software). Data are displayed as mean ± SD, box plots as median with 25^{th} or 75^{th} quantiles and min/max whiskers. Continuous data were tested for distribution and individual groups were tested by use of two-sided Chi-square test, unpaired t-test, unpaired Mann-Whitney-U-test, Kruskal-Wallis test, or paired Wilcoxon signed rank test, all performed as two-sided tests. If applicable adjustment for multiple testing was done. *P* values of < 0.05 were considered statistically significant.

### 2. Results

### Mass spectrometry-based immunopeptidomics uncovers the antigenic landscape of primary LPCs

To investigate the immunopeptidomic landscape of primary LPCs, the inventors first screened a cohort of 26 AML patients for the presence of CD34⁺CD38⁻LPCs within PBMCs revealing a low median frequency of 0.18% (range 0.00 - 40.6%). Subsequent LPC enrichment was performed for a sample panel (n = 11) exhibiting an appropriate frequency of LPCs. Thereby, the LPC frequency was significantly enriched from 5.27% (range 0.02 - 40.6%) in presorting to 92.1% (range 40.0 - 99.7%) in postsorting samples (LPCₑₙᵣ population, Figure 1A, B). The CD34⁺CD38⁻ LPCₑₙᵣ population of UPN01 showed *in vivo* leukemic engraftment of human CD33⁺ and CD33⁺CD117⁺ cells in the bone marrow, peripheral blood, spleen, and liver of NOD/SCID/IL2Rγ^{null} (NSG) mice (n = 4, Figure 1C). T cell-mediated tumor immunosurveillance requires sufficient HLA expression on tumor cells and especially on LPCs. Thus, the inventors quantified HLA surface expression on LPCs (n = 11) compared to CD34⁺CD38⁺ AML blasts (n = 11) and HV-derived CD34⁺ HPCs (n = 18, Figure 1D, E). HLA class I and HLA-DR surface expression of LPCs showed patient-individual heterogeneity, with molecule counts per cell ranging from 31,054 to 310,084 (median 81,874) for HLA class I and from 1,198 to 48,454 (median 10,007) for HLA-DR, respectively. AML blasts and HPCs showed comparable HLA class I surface expression with 76,333 and 123,567 HLA class I molecules per cell in median, respectively (Figure 1D). LPCs express lower, but not significantly decreased HLA-DR molecules compared to AML blasts (median 20,540) and HPCs (median 28,349, Figure 1E). Mass spectrometry-based analysis of the naturally presented immunopeptidome of LPCₑₙᵣ and autologous blast samples reveled a total of 16,342 (range 127 - 7,603, median 1,930) and 32,961 (range 1,151 - 10,489, median 5,588) unique HLA class I ligands identified from LPCₑₙᵣ samples and autologous blasts samples (n = 10), respectively (Figure 1F). Mapping the HLA class II immunopeptidomes of 11 LPCₑₙᵣ and autologous blast samples, we identified 16,638 (range 450 - 6,218, median 1,458) and 25,128 (range468 - 10,217, median 2,238) different HLA class II-presented peptides, respectively (Figure 1G).

Comparison of LPC- and AML blast-derived immunopeptidomes revealed a substantial overlap of HLA-presented peptides in LPC and autologous blast samples with 39.4% and 35.1% shared HLA class I and HLA class II peptides, respectively. Nevertheless, 6.8% and 18.7% of the total identified HLA class I and HLA class II peptides showed exclusive presentation of LPCs, respectively (Figure 1H). The nature of the identified HLA-presented peptides in terms of peptide length showed comparable distributions between LPCs and blasts with the expected length distributions for HLA class I- and HLA class II-presented peptides (Figure 1I). Analysis of the amino acid composition of HLA-presented peptides in total and position-specific revealed no substantial differences between LPC- and blast-derived immunopeptidomes (Figure 1J, Figure 2A, B).

Together, these data demonstrate that LPCs present HLA class I- and HLA class II-restricted antigens comparable but not identical to AML blasts highlighting the importance to select AML-associated antigen targets for immunotherapeutic approaches based on a combined approach including LPCs.

### Comparative immunopeptidomics profiling identifies LPC-associated HLA class I-restricted peptides

For the identification of novel AML-associated targets targeting not only AML blasts but also LPCs, we comprehensively mapped the HLA class I immunopeptidome of 47 primary AML samples (Table 5) including the above-described samples sorted for LPCs and autologous blasts.

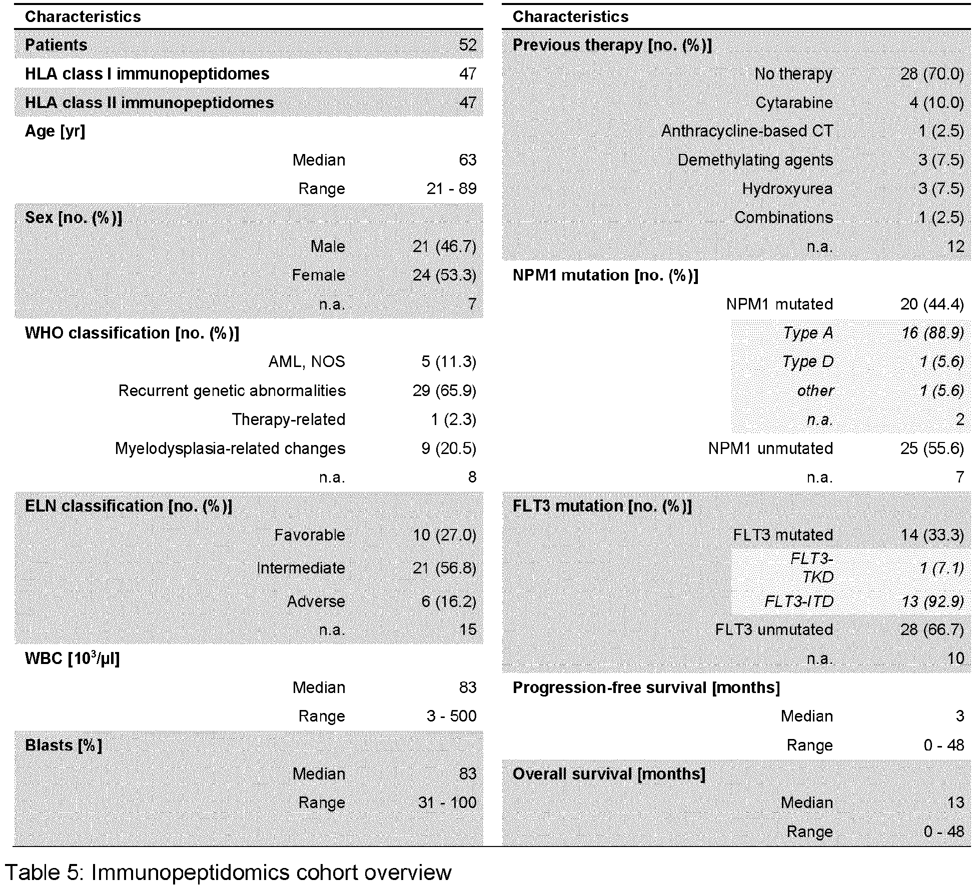

The inventors identified a total of 72,042 unique HLA class I ligands mapped to 10,609 source proteins, obtaining 97% of the estimated maximum attainable source protein coverage (Figure 3A). The number of identified peptides per patient ranged from 542 to 11,240 (median 3,143). For the identification of AML-associated antigen targets, the inventors performed comparative immunopeptidome profiling with a benign dataset (n = 332), which is comprised amongst others PBMC and CD34⁺ enriched HPC samples, which contains 72,129 unique HLA class I ligands. Overlap analysis revealed 13,019 AML-exclusive HLA class I ligands (Figure 3B) of which only one were identified on at least 20% of all analyzed samples (Figure 3C) due to the variety of different HLA class I allotypes covered within the AML dataset. In total, 48 different HLA class I allotypes were covered achieving a population coverage of 99.9% of the world population with at least one allotype (Figure 3D). Allotype frequencies ranged from 2.1% to 55.3% and where not significantly different for the majority of allotypes (72%, 39/54) compared to the benign dataset (Figure 4A).

For the identification of high frequent AML-associated HLA class I antigens, the inventors performed HLA allotype-specific comparative profiling (Figure 3E, Figure 4B) for the most common HLA class I allotypes HLA-A*01 (29.8% frequency in AML cohort), -A*02 (48.9%), -B*07 (25.5%), -B*08 (23.4%), and -C*07 (55.3%) achieving 71.1% of population coverage within the world population. This analysis revealed 48 high frequent and FDR-controlled HLA-A*01-, 28 -A*02-, 185 -B*07-, 161 -B*08-, and 11 -C*07-restricted AML-associated antigen targets to be frequently presented on allotype-matched samples with frequencies of at least 20% and up to 58% (Figure 3E, Figure 4B, C). An additional validation step includes an AML_{MR} immunopeptidomics dataset derived from PBMC samples of AML patients in molecular remission (n = 8) to prove antigen target presentation only on malignant cells. The majority of the defined AML-associated targets (85% for FLT3, 64% for NPM1) were identified independently of the FLT3 and NPM1 mutational status of the analyzed patient samples. To identify those AML-associated antigens which are also presented on LPCs, the inventors compared the total AML and benign datasets to the immunopeptidomics data of the LPCₑₙᵣ samples identifying 2,322 LPC/AML shared antigens presented on both AML blasts and LPCs but absent on any benign tissue including CD34⁺ enriched HPCs (Figure 3F). Of the previously identified AML-associated antigen targets 41.9% (179/434) are LPC/AML shared antigens (Figure 3G), which represent prime targets for the dual T cell-based targeting of AML blasts and LPCs.

### Identification of AML- and LPC-associated HLA class II-restricted antigens

For HLA class II, the inventors were able to identify a total of 61,205 unique peptides (range 590 - 10,733 per patient, median 2,168) originating from 5,922 source proteins and obtaining 85% of the estimated maximum attainable source protein coverage by analyzing 47 primary AML samples (Table 5, Figure 5A). The inventors utilized their previously established immunopeptidome profiling platform (Bilich, T. et al. The HLA ligandome landscape of chronic myeloid leukemia delineates novel T-cell epitopes for immunotherapy. Regular Article IMMUNOBIOLOGY AND IMMUNOTHERAPY (2019)) delineating 3 groups of antigens: peptide targets, protein targets, and hotspot targets. First, overlap analysis and comparative profiling with a benign dataset (n = 312) at peptide level revealed 10,931 AML-exclusive HLA class II-restricted peptides (Figure 5B) of which 5 are FDR-controlled presented on at least 15% of samples and do not have length variants on benign tissue samples (peptide targets, Figure 5C, Figure 6A). Second, HLA peptide source protein profiling revealed 311 AML-exclusive proteins, of which CCL23 and RRS1 show frequent and FDR-controlled AML-associated HLA-restricted presentation with 7 and 2 proteotypic peptides (Figure 6B-D). As a third group, the inventors analyzed AML-exclusive hotspots by peptide clustering, which identified 5 AML-associated hotspots with a representation frequency of at least 15% within the proteins FLT3, IL1AP, HPRT, KIT, and AP2B1 (Figure 5D, Figure 6E). In a next step, the inventors screened their novel identified AML-associated peptide, protein, and hotspot targets for their presentation on LPCₑₙᵣ samples (Figure 5E, F). Similar to HLA class I, the majority of identified HLA class II-restricted targets (64% for FLT3, 50% for NPM1) showed no dependence on the FLT3 and NPM1 mutation status of the analyzed patient samples. Of the AML-associated HLA class II peptide, protein and hotspot targets 66.7% (8/12) are LPC/AML shared antigens (Figure 5G), which represent prime targets for the dual T cell-based targeting of AML blasts and LPCs. Furthermore, we screened our HLA class II immunopeptidome dataset for the present of high frequent LPC-exclusive and LPC-associated peptide and protein antigen targets (Figure 5H, Figure 6F), identifying further interesting candidates for specific LPC immune targeting.

### The role of neoantigens and cryptic peptides in AML

In addition to the definition of novel AML- and LPC-associated antigens, the inventors screened their AML immunopeptidomics cohort for naturally presented mutation-derived neoepitopes from common mutations. Thereby, they were able to identify and validate the presentation of two NPM1 mutation-derived HLA-A*11- and HLA-A*03-restricted peptides P16_{A*11_mut} (AVEEVSLRK; SEQ ID NO: 16) and P17_{A*03_mut} (LAVEEVSLR; SEQ ID NO: 17) as well as the IDH2 R140Q mutation-derived HLA class II-presented neoepitope P15_{II_mut} (KLKKMWKSPNGTIQNILGGTVF; SEQ ID NO: 1) (Figure 5I, Figure 7, Figure 8). Besides the classical neoepitopes derived from mutations, cryptic peptides that originate from non-coding regions such as 5'- and 3'- untranslated region (UTR), non-coding RNAs (ncRNA), intronic and intergenic regions, or shifted reading frames in annotated protein coding regions (off-frame) came into focus as a new potential source of tumor-associated antigens. The inventors analyzed the AML and benign immunopeptidomics cohorts with Peptide-PRISM and identified 623 AML-associated HLA class I-presented cryptic peptides mainly derived from UTR5 and off-frame regions that were never identified on any benign tissue (Figure 9A). Of these, 109 peptides in total were identified on LPCₑₙᵣ samples with 26 LPC-exclusive peptides (Figure 9B) mainly originating from off-frame regions (Figure 9C). The inventors selected the high frequently (26% and 42% allotype-specific) presented peptides P2_cry_{A*02} (ILLSPPLLTI; SEQ ID NO: 40) and P2_cry_{B*07} (GPDDGRGVL; SEQ ID NO: 41) for further validation and characterization. They are derived from the UTR5 region of CHRFAM7A and off-frame from TSPAN2 (Figure 9D). The mass spectrometric identification was validated using isotope-labeled synthetic peptides (Figure 9E). The identified neoepitopes and cryptic peptides further expand the panel of novel AML-associated antigen targets.

### Novel AML/LPC-associated antigens exhibited pre-existing and de novo inducible immune responses in AML patients and HVs

For further characterization and immunogenicity analyses, the inventors selected a panel of high frequent HLA class I- and HLA class II-presented peptides including neoepitopes, cryptic peptides and AML-associated tumor antigens that are presented on LPCs and restricted to common HLA allotypes (Table 6).

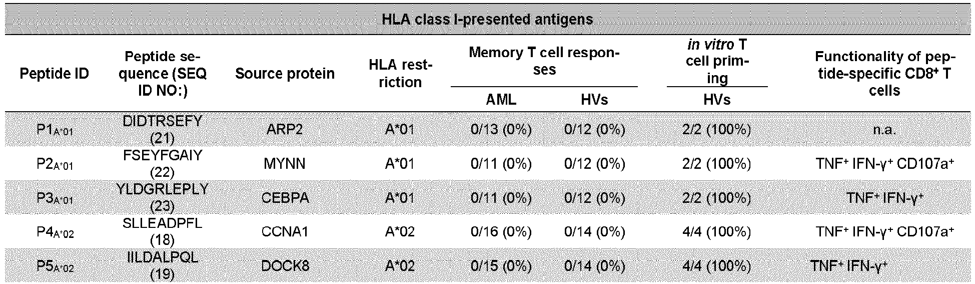

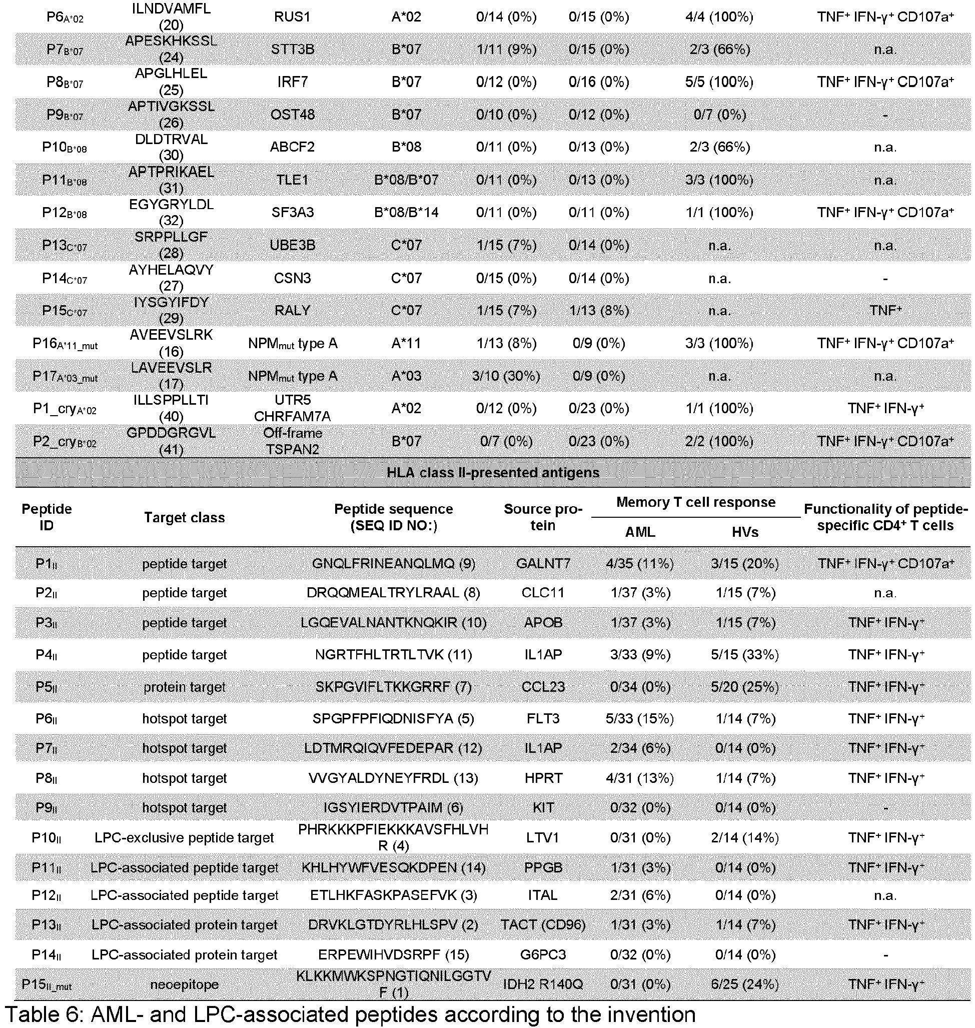

Experimentally acquired spectra of the selected peptide identifications were validated by comparison of mass spectrometric fragment spectra using isotope-la-beled synthetic peptides (Figure 9E, Figure 7, Figure 8). To detect preexisting memory T cell responses against the selected HLA class I-restricted peptides, the inventors performed IFN-γ ELISpot assays using HLA-matched PBMCs from HVs and AML patients (Figure 10A). They observed IFN-γ secretion for 5/19 HLA class I peptides in up to 30% of AML patient samples and for 1/19 peptides in up to 8% of HV samples (Table 2), however these immune responses were mainly mediated by CD4⁺ T cells (Figure 10B). Using aAPC-based *in vitro* priming of naive CD8⁺ T cells of HLA-matched HVs, the inventors confirmed *de novo* induction and effective expansion of antigen-specific CD8⁺ T cells for 12/13 investigated HLA class I peptides in at least 66% of analyzed HV samples (Figure 10C, Table 2). Induced T cells produce the cytokines IFN-γ and TNF as well as the degranulation marker CD107a upon peptide stimulation (Figure 10D). Notably, peptide-specific T cell responses could even be induced *de novo* in AML patient samples that had not shown preexisting immune responses (Figure 10E). Furthermore, polyclonal effector cells comprising 4.0% P16_{A*11_mut}-specific T cells exhibited a peptide-specific cell lysis of 82% of peptide-loaded autologous cells, whereas a non-specific effector cell population revealed 3.4% relative lysis of the same targets. The specific lysis showed effector-to-target ratio dependent characteristics with specific lysis decreasing with reduced effector-to-target ratios (Figure 10F). In total, 89.5% (17/19) of the selected HLA class I-restricted peptides were proven to be immunogenic, with 26.3% (5/19) showing preexisting memory T cells in AML patients or HVs (Figure 10G).

The functional characterization of HLA class II-restricted peptides using IFN-γ ELISpot assay (Figure 11A) and intracellular cytokine staining (Figure 11B) reveled strong preexisting CD4⁺ T cell-mediated immune responses in AML patients or HVs against 86.7% (13/15) peptides with frequencies up to 33% (Figure 11C, D, Table 2). Interestingly, the source protein CCL23 of the peptide P5₁₁ (SKPGVIFLTKKGRRF) and the peptide itself showed very high structural, sequence and physiochemical property similarities with the viral protein VMI2 of the human herpes virus 8 (Figure 12A, B, C). Although, P5_{II}-specific T cell responses detected in PBMC samples of HVs and AML patients are P5_{II}-specific and do not exhibited cross-reactivity to the viral peptide (Figure 12D). The FLT3-derived peptide P6_{II} (SPGPFPFIQDNISFYA) elicited in a single patient a CD8⁺ T cell-mediated immune response (Figure 12E, F). *In silico* prediction with the patient's HLA allotype reveled four possible HLA class I-restricted peptides to be embedded in the long sequence (Figure 13G). Longitudinal assessment of peptide-specific T cell response after allogenic stem cell transplantation in a single patient (Figure 13A) using IFN-γ ELISpot assay reveled an increasing response over time starting 17 months after transplantation (Figure 13B, C). At month 12 after transplantation no peptide-specific T cell response could be detected, which might be due to a general immune suppression caused by pred-nisolone treatment at that time.

### Diverse antigen presentation and immune recognition of AML-associated antigens are associated with improved survival of AML patients

As a last step, the inventors investigated the prognostic relevance of AML-exclusive antigen presentation and peptide-specific immune recognition. The presentation of AML-exclusive antigens, in terms of different presented peptides called the immuno-peptidome diversity, did not differ significantly according to demographics and tumor characteristics, including age, sex, ELN, karyotype, FLT3-ITD and NPM1 mutation. Performing survival analysis, the inventors could not observe an impact of HLA class I immunopepti-dome diversity on treatment failure-free (FFS) and overall (OS) survival (Figure 14A, B). Whereas for HLA class II-restricted peptide diversity, they observed a significantly improved OS (Figure 11E), but not FFS (Figure 14C) for those patients presenting higher numbers of different peptides, highlighting the importance of CD4⁺ immune responses for tumor immunosurveillance. The immune recognition of AML- and LPC-associated antigens in AML patients (n = 56), *i.e.,* the occurrence of preexisting immune responses against HLA class II-restricted AML- and LPC-associated antigens as detected by IFN-γ ELISpot assays revealed a strong prognostic impact on FFS but not on OS (Figure 11F). However, this cohort is a highly selected patient cohort as only patients, who survived long enough were included and differences in disease stage, cytogenetics or preceding treatments including stem cell transplantation were not taken into account. Therefore, the inventors performed a subgroup analysis in patients after allogenic stem cell transplantation, pointing towards, without reaching statistically significance, the importance of antigen-specific T cell responses for immune surveillance (Figure 14D).

## Claims

1. A pharmaceutical composition for the diagnosis and/or prevention and/or treatment of hematological neoplasms comprising at least one peptide which binds to class II molecules of the major histocompatibility complex (MHC class II molecules) and/or induces T cells cross-reacting with said peptide, and a pharmaceutically acceptable carrier, wherein said at least one peptide comprises an amino acid sequence which is selected from the group consisting of: SEQ ID NO: 1 to SEQ ID NO: 15 and variant sequences thereof which are at least 88% homologous to SEQ ID NO: 1 to SEQ ID NO: 15, wherein said peptide is not a full-length polypeptide.

2. The pharmaceutical composition according to claim 1, wherein it comprises at least 2, preferably at least 3, further preferably at least 4, further preferably at least 5, further preferably at least 6, further preferably at least 7, further preferably at least 8, further preferably at least 9, and highly preferably at least 10 different peptides, each peptide comprising an amino acid sequence which is selected from the group consisting of: SEQ ID NO: 1 to SEQ ID NO: 15 and variant sequences thereof which are at least 88% homologous to SEQ ID NO: 1 to SEQ ID NO: 15, wherein said peptide is not a full-length polypeptide.

3. The pharmaceutical composition according to claim 1 and 2, wherein it comprises different peptides comprising the following amino acid sequences:
KLKKMWKSPNGTIQNILGGTVF (SEQ ID NO: 1)
DRVKLGTDYRLHLSPV (SEQ ID NO: 2)
ETLHKFASKPASEFVK (SEQ ID NO: 3)
PHRKKKPFIEKKKAVSFHLVHR (SEQ ID NO: 4)
SPGPFPFIQDNISFYA (SEQ ID NO: 5)
IGSYIERDVTPAIM (SEQ ID NO: 6)
SKPGVIFLTKKGRRF (SEQ ID NO: 7)
DRQQMEALTRYLRAAL (SEQ ID NO: 8)
GNQLFRINEANQLMQ (SEQ ID NO: 9)
LGQEVALNANTKNQKIR (SEQ ID NO: 10)
NGRTFHLTRTLTVK (SEQ ID NO: 11)
LDTMRQIQVFEDEPAR (SEQ ID NO: 12)
VVGYALDYNEYFRDL (SEQ ID NO: 13)
KHLHYWFVESQKDPEN (SEQ ID NO: 14)
ERPEWIHVDSRPF (SEQ ID NO: 15).

4. The pharmaceutical composition according to any of the preceding claims, wherein it comprises at least one additional peptide which binds to class I molecules of the major histocompatibility complex (MHC class I molecules) and/or induces T cells cross-reacting with said peptide, wherein said at least one additional peptide comprises an amino acid sequence which is selected from the group consisting of: SEQ ID NO: 16 to SEQ ID NO: 32 and variant sequences thereof which are at least 88% homologous to SEQ ID NO: 16 to SEQ ID NO: 32, wherein said peptide is not a full-length polypeptide.

5. The pharmaceutical composition according to claim 4, wherein the at least one additional peptide is selected depending on the MHC class I allotype of the individual to be treated, preferably the at least one additional peptide is selected as follows:
| MHC class I allotype | Amino acid sequence of peptide |
|---|---|
| A*11 | SEQ ID NO: 16 |
| A*03 | SEQ ID NO: 17 |
| A*02 | SEQ ID NO: 18 to SEA ID NO: 20 |
| A*01 | SEQ ID NO: 21 to SEQ ID NO: 23 |
| B*07 | SEQ ID NO: 24 to SEQ ID NO: 26 |
| C*07 | SEQ ID NO: 27 to SEQ ID NO: 29 |
| B*08 | SEQ ID NO: 30 to SEQ ID NO: 32 |
, wherein the amino acid sequences of each MHC class I allotype group includes variant sequences which are at least 88% homologous.

6. The pharmaceutical composition according to claim 4 or 5, wherein it comprises at least 2, preferably at least 3, further preferably at least 4, further preferably at least 5, and highly preferably at least 6 different additional peptides, each additional peptide comprising an amino acid sequence which is selected from the group consisting of: SEQ ID NO: 16 to SEQ ID NO: 32 and variant sequences thereof which are at least 88% homologous to SEQ ID NO: 16 to SEQ ID NO: 32, wherein said additional peptide is not a full-length polypeptide.

7. The pharmaceutical composition according to any of the preceding claims which is a vaccine, preferably a vaccine against hematological neoplasms, further preferably a vaccine against acute myeloid leukemia (AML).

8. The pharmaceutical composition according to any of the preceding claims further comprising an adjuvant, preferably XS15, further preferably XS15 dissolved in montanide.

9. A peptide comprising an amino acid sequence selected from the group consisting of SEQ ID NO: 1 to SEQ ID NO: 32, and variant sequences thereof which are at least 88% homologous to SEQ ID NO: 1 to SEQ ID NO: 32, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide,
preferably
said peptide has the ability to bind to an MHC class I or II molecule, and wherein said peptide, when bound to said MHC, is capable of being recognized by CD4 and/or CD8 T cells,
further preferably
the amino acid sequence thereof comprises a continuous stretch of amino acids according to any one of SEQ ID NO: 1 to SEQ ID NO: 32.

10. A nucleic acid, encoding for a peptide or variant thereof according to claim 9, optionally linked to a heterologous promoter sequence, or an expression vector expressing said nucleic acid.

11. A recombinant host cell comprising the peptide according to claim 9 or the nucleic acid or the expression vector according to claim 10, wherein said host cell preferably is selected from an antigen presenting cell, such as a dendritic cell, a T cell or an NK cell.

12. An *in vitro* method for producing activated T lymphocytes, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to claim 9.

13. An activated T lymphocyte, produced by the method according to claim 16, that selectively recognizes a cell which presents a polypeptide comprising an amino acid sequence given in claim 9.

14. A kit comprising:
(a) a container comprising the pharmaceutical composition according to any one of claims 1-8, the peptide(s) according to claim 9, the nucleic acid or the expression vector according to claim 10, the recombinant host cell according to claim 11 or the activated T lymphocyte according to claim 13, in solution or in lyophilized formulation;
(b) optionally, a second container containing a diluent or reconstituting solution for the lyophilized formulation;
(c) optionally, instructions for (i) use of the solution or (ii) reconstitution and/or use of the lyophilized formulation.

15. A method for producing a personalized anti-cancer vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or over-presentation in tumors as compared to normal tissues;
c) selecting at least one peptide from the warehouse that matched a TUMAP identified in said patient; and
d) formulating the personalized vaccine based on step c),
wherein said warehouse comprises a plurality of peptides and/or variant sequences according to claim 9.
